# EUROPEAN PATENT APPLICATION

(11) **EP 3 757 209 A1**
(43) Date of publication of application: **30.12.2020**
(21) Application number: 19182814.4
(22) Date of filing: 27.06.2019
(51) Int. Cl.: C12N 9/10, C12N 9/24, C12P 19/04, C12P 19/14, C12P 19/18, A61K 31/733, A23L 33/125, A23L 29/30

(54) **ENZYMATIC PRODUCTION OF LEVAN-BASED, PREBIOTIC FRUCTOOLIGOSACCHARIDES**

(71) Applicant: Rheinische Friedrich-Wilhelms-Universität Bonn, 53113 Bonn (DE)
(72) Inventor: DEPPENMEIER, Uwe, 53797 Lohmar (DE); HÖVELS, Marcel, 53121 Bonn (DE); KOSCIOW, Konrad, 53111 Bonn (DE)
(74) Representative: Viering, Jentschura & Partner mbB Patent- und Rechtsanwälte

(57) **Abstract**

The present invention relates to a method for preparing (levan-based) fructooligosaccharides (FOS) using at least one (genetically modified) host organism. Through the production of a levansucrase and an endolevanase in the host organism, the enzymes can be used to convert the substrate sucrose into FOS. Additionally, the invention is directed to fructooligosaccharides obtainable by the method according to the invention; a specific expression vector, a specific genetically modified host organism, and a specific cell extract or culture supernatant, which are usable for the production of FOS; and a prebiotic or food supplement comprising or consisting of the FOS. Finally, the invention relates to a method for preparing levan using a levansucrase.

## Description

### FIELD OF THE INVENTION

The present invention relates to a method for preparing levan or levan-based fructooligosaccharides (FOS) using at least one (genetically modified) host organism or enzymes recombinantly expressed in such host organism. Specifically, the expression of a levansucrase and/or an endolevanase in the host organism allows to convert the substrate sucrose into levan and/or FOS. Additionally, the invention is directed to levan and/or fructooligosaccharides obtainable by the method according to the invention; a specific expression vector, a specific genetically modified host organism, and a specific cell extract or culture supernatant, which are usable for the production of levan and/or FOS; and a prebiotic or food supplement comprising or consisting of the levan and/or FOS.

### BACKGROUND OF THE INVENTION

Levan is a polymer of β-2,6-glycosidically linked fructose units. The structural formula of a monomeric fructose unit of the levan backbone is shown below:

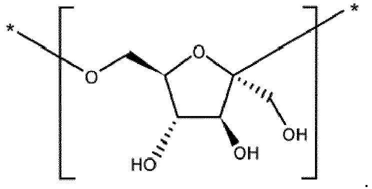

The synthesis of levan is catalyzed by levansucrases (EC 2.4.1.10), which are produced by numerous microbial species and occasionally also by plant species (Oner et al. (2016) Biotechnol. Adv. 34:827-844). The substrate for the enzymatic reaction is preferably the disaccharide sucrose, which is hydrolyzed in an initial step into the monomer subunits glucose and fructose. While the glucose is released from the active center, the remaining fructose is coupled to a new sucrose molecule. This process, in which the sucrose serves as the terminal acceptor, can be repeated cyclically, whereby fructose units from the sucrose are successively added to the growing levan chain. This can result in degrees of polymerization of up to 100,000 fructose units, which corresponds to a molecular weight of over 100 tons mol⁻¹ (Tanaka et al. (1980) J. Biochem. 87:297-303). Due to its high molecular weight and the associated physicochemical properties, levan cannot or only very poorly be purified from corresponding process solutions by filtration or chromatographic methods. Industrial production of this promising prebiotic has therefore not yet been realized.

A prebiotic is defined as "a substrate that is selectively utilized by host microorganisms conferring a health benefit" (Gibson et al. (2017) Nat. Rev. Gastroenterol. Hepatol). In earlier definitions, selectivity mostly referred to members of the genera *Lactobacillus* and *Bifidobacterium.* Especially the specific stimulation of bifidobacteria (bifidogenesis) was considered to have a prebiotic effect. Meanwhile, further representatives of intestinal microbiota have been identified, which mediate positive effects on host health. These organisms are usually butyrate producers of the clostridial clusters IV (*Faecalibacterium*) and XIVa (*Anaerostipes, Eubacterium* & *Roseburia*), which are stimulated by intestinal cross-feeding (Barcenilla et al. (2000) Appl. Environ. Microbiol., doi: 10.1128/AEM.66.4.1654-1661.2000; Schwiertz et al. (2002) Syst. Appl. Microbiol., doi: 10.1078/0723-2020-00096; Duncan et al. (2004) Appl. Environ. Microbiol., doi: 10.1128/AEM.70.10.5810-5817.2004; Belenguer et al. (2006) Appl. Environ. Microbiol., doi: 10.1128/AEM.72.5.3593-3599.2006; Falony et al. (2009) Appl. Environ. Microbiol., doi: 10.1128/AEM.01488-08; Belenguer et al. (2011) FEMS Microbiol. Ecol., doi: 10.1111/j.1574-6941.2011.01086.x). The health-promoting effect of prebiotics is based on the metabolic products generated by the selectively stimulated host microorganisms. These products are the short chain fatty acids (SCFAs) acetate (C2 body), propionate (C3) and *n*-butyrate (C4). The physiological effects of these SCFAs on the local and systemic level are versatile and influence, among others, the functionality of colonocytes, intestinal homeostasis, the immune system, composition and number of blood lipids, appetite and renal physiology (Roberfroid et al. (2010) Br. J. Nutr. 104 Suppl:S1-63, doi: 10.1017/S0007114510003363; O'Keefe (2016) Nat. Rev. Gastroenterol. Hepatol. 13:691-706, doi: 10.1038/nrgastro.2016.165; Pluznick (2016) Kidney Int. 90:1191-1198). The relationship between structure and function of the microbial composition, the use of prebiotics and host health has gained importance in recent years through numerous publications (Gibson (2010) Food Sci. Technol. Bull. Funct. Foods 7:1-19, doi: 10.1616/1476-2137.15880; Rastall et al. (2015) Curr. Opin. Biotechnol.; Hutkins et al. (2016) Curr. Opin. Biotechnol. 37:1-7, doi: 10.1016/j.copbio.2015.09.001). This development also has an impact on the corresponding industrial and economic sectors. For example, the market for prebiotic ingredients was valued at USD 3.65 billion in 2016. This value is expected to double to more than USD 7.3 billion by 2023, with an average annual growth of 10.4 %.

Currently, the market for prebiotic ingredients is dominated by three compounds that officially have a prebiotic status: Inulin, galactooligosaccharides and lactulose. Promising substances that have not yet been classified as prebiotics and are therefore not produced and marketed on an industrial scale are isomaltooligosaccharides, lactosucrose and xylooligosaccharides.

The fructose-based polymer levan is also considered a promising prebiotic. As has been verified in numerous *in vivo* studies (Jang et al. (2003) J. Microbiol. Biotechnol.; Hamdy et al. (2018) Biocatal. Agric. Biotechnol., doi: 10.1016/j.bcab.2017.12.001) and *in vitro* (Porras-Domínguez et al. (2014) Process Biochem., doi: 10.1016/j.procbio.2014.02.005; Adamberg (2015) PLoS One., doi: 10.1371/journal.pone.0144042), levan can be selectively fermented by beneficial representatives of our intestinal flora and thus has a beneficial effect on host health.

Levan has unique physicochemical properties, including antioxidant (Liu et al. (2012) Food Chem. Toxicol., doi: 10.1016/j.fct.2011.11.016), anti-inflammatory (Srikanth et al. (2015) Carbohydr. Polym., doi:10.1016/j.carbpol.2014.12.079), antibacterial (Byun et al. (2014) Int. J. Food. Sci. Technol., doi: 10.1111/ijfs.12304) and antiviral (Esawy et al. (2011) Carbohydr. Polym., doi: 10.1016/j.carbpol.2011.05.035) functions. Due to its versatility, the polymer could compete with established compounds in the fast-growing market for prebiotic ingredients. However, due to the high degree of polymerization, levan cannot be purified by filtration or chromatographic methods. For this reason, no industrial production of levan-based dietary fiber has yet been realized.

Therefore, novel processes for efficient and/or large-scale production of levan-based fructooligosaccharides (FOS) are needed.

Surprisingly, the inventors were able to develop a method to circumvent the problematic and labor-intensive purification of high-molecular levan chains. The combined activity of two enzymes (levansucrase and endolevanase), in particular the levansucrase from *G. japonicus* LMG 1417 and the endolevanase from *Azotobacter* (*A.*) *chroococcum* DSM 2286 which are newly characterized, allows the production of (short-chain) fructooligosaccharides based on levan, preferably by starting from the renewable and low-cost substrate sucrose. In contrast to the polymer form, the (short-chain) FOS can be easily purified from industrial process solutions, e.g. by using suitable filtration systems.

### SUMMARY OF THE INVENTION

In a first aspect, the invention relates to a method for preparing fructooligosaccharides (FOS) comprising or consisting of the following steps:
(a) optionally introducing at least one nucleic acid molecule comprising a first nucleotide sequence which encodes a levansucrase and/or a second nucleotide sequence which encodes an endolevanase into at least one host organism;
(b) providing
   (i) a host organism comprising a first nucleotide sequence which encodes a levansucrase and a second nucleotide sequence which encodes an endolevanase;
      or
   (ii) a first host organism comprising a first nucleotide sequence which encodes a levansucrase and a second host organism comprising a second nucleotide sequence which encodes an endolevanase;
(c) cultivating
   (i) the host organism comprising the first nucleotide sequence which encodes a levansucrase and the second nucleotide sequence which encodes an endolevanase under conditions which allow the expression of the first and the second nucleotide sequence;
      or
   (ii) the first host organism comprising the first nucleotide sequence which encodes a levansucrase and the second host organism comprising the second nucleotide sequence which encodes an endolevanase under conditions which allow the expression of the first and the second nucleotide sequence;and
(d) preparing fructooligosaccharides using the levansucrase and the endolevanase expressed in step (c), by subjecting the enzymes to conditions which allow the production of the fructooligosaccharides.

In a preferred embodiment, in step (d) the levansucrase and the endolevanase are, individually or together,
(i) comprised in a culture supernatant from the culture medium used in step (c), wherein the culture supernatant is subjected to conditions which allow the preparation of said fructooligosaccharides; and/or
(ii) comprised in a cell extract from the at least one host organism cultivated in step (c), wherein the cell extract is subjected to conditions which allow the preparation of said fructooligosaccharides; and/or
(iii) comprised in a host organism or at the surface of the host organism cultivated in step (c), wherein the host organism is subjected to conditions which allow the preparation of said fructooligosaccharides; and/or
(iv) provided in a purified or immobilized form.

In various embodiments, the method according to the invention comprises or consists of the following steps:
(a) optionally introducing one nucleic acid molecule comprising a first nucleotide sequence which encodes a levansucrase and a second nucleotide sequence which encodes an endolevanase into a host organism;
(b) providing a host organism comprising a first nucleotide sequence which encodes a levansucrase and a second nucleotide sequence which encodes an endolevanase;
(c) cultivating the host organism comprising the first nucleotide sequence which encodes a levansucrase and the second nucleotide sequence which encodes an endolevanase under conditions which allow the expression of the first and the second nucleotide sequence; and
(d) using the levansucrase and the endolevanase obtained in step (c) and comprised in the cell extract from the host organism or in the culture supernatant from the culture medium after step (c), and subjecting said cell extract or culture supernatant to conditions which allow the preparation of said fructooligosaccharides.

Preferably, the fructooligosaccharides obtained in step (d) comprise, essentially consist of or consist of compounds of the formulas Fₘ and/or GFₙ, wherein
F is a monomeric fructose unit, preferably D-fructose unit;
G is a monomeric glucose unit, preferably D-glucose unit;
m is ≥3, preferably 3 to 20, more preferably 3 to 15, more preferably 3 to 13, most preferably 3 to 10;
n is ≥2, preferably 2 to 19, more preferably 2 to 14, more preferably 2 to 12, most preferably 2 to 9;
m and/or n are the same or different in the individual fructooligosaccharides obtained in step (d); and the fructose units are covalently coupled to each other by β-(2→-6) linkages and may further comprise β-(2→1) branching. The fructooligosaccharides obtained in step (d) of the method for preparing fructooligosaccharides according to the invention are preferably also referred to as short-chain fructooligosaccharides.

In various embodiments, the (amino acid sequence of the) levansucrase comprises or consists of
(i) an amino acid sequence set forth in SEQ ID Nos. 1 or 2; or
(ii) an amino acid sequence, which has at least 60 %, 70 %, 75 %, 80 %, 85 %, 90 %, 91 %, 92 %, 93 %, 94 %, 95 %, 96 %, 97 %, 98 % or 99 % sequence identity with the amino acid sequence set forth in SEQ ID Nos. 1 or 2 over the full length of the sequence.

In various embodiments, the levansucrase comprises or consists of the amino acid sequence of SEQ ID NO:2 but lacks the N-terminal M of SEQ ID NO:2. Also comprised are fragments of the above-described levansucrases that retain at least 75 % of the activity of the full length sequence but lack one or more N-terminal and/or C-terminal amino acids.

In various embodiments, the (amino acid sequence of the) endolevanase comprises or consists of
(i) an amino acid sequence set forth in SEQ ID Nos. 5 or 6; or
(ii) an amino acid sequence, which has at least 60 %, 70 %, 75 %, 80 %, 85 %, 90 %, 91 %, 92 %, 93 %, 94 %, 95 %, 96 %, 97 %, 98 % or 99 % sequence identity with the amino acid sequence set forth in SEQ ID Nos. 5 or 6 over the full length of the sequence.

In various embodiments, the endolevanase comprises or consists of the amino acid sequence of SEQ ID NO:6 but lacks the first 35 N-terminal amino acids of SEQ ID NO:6, i.e. starts with A36. Accordingly, also comprised are fragments of the above-described endolevanase that retain at least 75 % of the activity of the full length sequence but lack one or more N-terminal and/or C-terminal amino acids.

In various embodiments, the first nucleotide sequence which encodes the levansucrase comprises or consists of
(i) a nucleotide sequence set forth in SEQ ID Nos. 3 or 4; or
(ii) a nucleotide sequence which has at least 70 %, 75 %, 80 %, 85 %, 90 %, 91 %, 92 %, 93 %, 94 %, 95 %, 96 %, 97 %, 98 % or 99 % sequence identity with the nucleotide sequence set forth in SEQ ID Nos. 3 or 4 over the full length of the sequence.

In various embodiments, these nucleotide sequences encode levansucrases that comprise or consist of the amino acid sequence set forth in SEQ ID Nos. 1 or 2 or an amino acid sequence which has at least 60 %, 70 %, 75 %, 80 %, 85 %, 90 %, 91 %, 92 %, 93 %, 94 %, 95 %, 96 %, 97 %, 98 % or 99 % sequence identity with the amino acid sequence set forth in SEQ ID Nos. 1 or 2 over the full length of the sequence or fragments thereof, as defined above.

In various embodiments, the second nucleotide sequence which encodes the endolevanase comprises or consists of
(i) a nucleotide sequence set forth in SEQ ID Nos. 7 or 8; or
(ii) a nucleotide sequence which has at least 70 %, 75 %, 80 %, 85 %, 90 %, 91 %, 92 %, 93 %, 94 %, 95 %, 96 %, 97 %, 98 % or 99 % sequence identity with the nucleotide sequence set forth in SEQ ID Nos. 7 or 8 over the full length of the sequence.

In various embodiments, these nucleotide sequences encode endolevanases that comprise or consist of the amino acid sequence set forth in SEQ ID Nos. 5 or 6 or an amino acid sequence which has at least 60 %, 70 %, 75 %, 80 %, 85 %, 90 %, 91 %, 92 %, 93 %, 94 %, 95 %, 96 %, 97 %, 98 % or 99 % sequence identity with the amino acid sequence set forth in SEQ ID Nos. 5 or 6 over the full length of the sequence or fragments thereof, as defined above.

Preferably, the conditions which allow the preparation of the fructooligosaccharides comprise providing sucrose to the levansucrase and endolevanase used in step (d).

In various embodiments, the sucrose is converted to the fructooligosaccharides obtained in step (d), wherein the conversion is catalyzed by the levansucrase and the endolevanase.

Preferably, the levansucrase hydrolyzes the sucrose and uses the released fructose to form fructan-polymers, also referred to as levan, preferably with up to 100.000 fructose units, and then the endolevanase hydrolyzes the fructan-polymers to prepare the fructooligosaccharides obtained in step (d).

In various embodiments, the method comprises after step (c) and before step (d) a further step to separate the culture medium from the cells (e.g. by centrifugation). Additionally, a further step of cell disruption and/or filtration and/or purification and/or immobilization and/or lyophilization, without being limited to these methods, can be present between step (c) and (d) of the method according to the invention.

In various embodiments, the method comprises after step (d) a further step (e) for purifying the fructooligosaccharides obtained in step (d).

The purification step (e) may be a chromatography step or a filtration step, without being limited to these methods.

Preferably, the at least one host organism, for example the first host organism and/or the second host organism, is a bacterial or yeast organism, preferably a bacterial organism, for example an *Escherichia coli* or a *Gluconobacter, Lactobacillus, Bifidobacterium, Zymomonas, Bacillus, Rahnella, Leuconostoc, Acetobacter, Azotobacter* or *Erwinia* species, preferably *Escherichia coli* or an *Azotobacter, Gluconobacter, Lactobacillus or Bifidobacterium* species, more preferably *an Escherichia coli* or an *Azotobacter* (e.g. *Azotobacter chroococcum,* in particular *Azotobacter chroococcum* DSM 2286) or *Gluconobacter species,* more preferably an *Escherichia coli* or a *Gluconobacter* species, such as *Gluconobacter japonicus, Gluconobacter cerinus* or *Gluconobacter oxydans,* in particular *Gluconobacter japonicus.* In some embodiments, the host organism may be *Escherichia coli* BL21 or *E. coli* BL21 DE3, or *Gluconobacter japonicus* LMG 1417. Suitable yeast organisms include, but are not limited to those of the genus *Saccharomyces* and *Pichia,* such as *Saccharomyces cerevisiae* and *Pichia pastoris.*

In preferred embodiments, the at least one nucleic acid molecule is an expression vector.

In a preferred embodiment, the first nucleotide sequence which encodes the levansucrase and/or the second nucleotide sequence which encodes the endolevanase is
(i) comprised in the expression vector; and/or
(ii) integrated in the genome, preferably in the chromosomal DNA, of the host organism.

In a second aspect, the invention relates to fructooligosaccharides obtainable by the method according to the invention.

In a third aspect, the invention relates to an expression vector comprising a first nucleotide sequence which encodes a levansucrase and/or a second nucleotide sequence which encodes an endolevanase; wherein the first nucleotide sequence which encodes the levansucrase comprises or consists of a nucleotide sequence
(i) set forth in SEQ ID Nos. 3 or 4; or
(ii) which has at least 70 %, 75 %, 80 %, 85 %, 90 %, 91 %, 92 %, 93 %, 94 %, 95 %, 96 %, 97 %, 98 % or 99 % sequence identity with the nucleotide sequence set forth in SEQ ID Nos. 3 or 4 over the full length of the sequence; and/or
wherein the second nucleotide sequence, which encodes the endolevanase, comprises or consists of a nucleotide sequence
(i) set forth in SEQ ID Nos. 7 or 8; or
(ii) which has at least 70 %, 75 %, 80 %, 85 %, 90 %, 91 %, 92 %, 93 %, 94 %, 95 %, 96 %, 97 %, 98 % or 99 % sequence identity with the nucleotide sequence set forth in SEQ ID Nos. 7 or 8 over the full length of the sequence.

In a fourth aspect, the invention relates to a genetically modified host organism comprising
(i) the expression vector as defined in the third aspect; and/or
(ii) the first nucleotide sequence which encodes the levansucrase and/or the second nucleotide sequence which encodes the endolevanase according to the invention in its genome, preferably in its chromosomal DNA; and/or
(iii) the levansucrase and/or the endolevanase according to the invention.

In a fifth aspect, the invention relates to a cell extract or culture supernatant comprising the levansucrase and/or the endolevanase according to the invention.

In a sixth aspect, the invention relates to a prebiotic or food supplement comprising or (essentially) consisting of the fructooligosaccharides according to the present invention.

Preferably, the prebiotic or food supplement comprising or consisting of the fructooligosaccharides obtainable by the method for preparing fructooligosaccharides according to the invention can be comprised, without being limited to it, in general food products, baby food and animal food.

In a seventh aspect, the invention relates to a method for preparing levan comprising or consisting of the following steps:
(a) optionally introducing at least one nucleic acid molecule comprising a first nucleotide sequence which encodes a levansucrase into a host organism;
(b) providing a host organism comprising a first nucleotide sequence which encodes a levansucrase;
(c) cultivating the host organism under conditions which allow the expression of the first nucleotide sequence;and
(d) using the levansucrase obtained in step (c) and subjecting it to conditions which allow the preparation of levan.

In a preferred embodiment, the levansucrase is
(i) comprised in a culture supernatant from the culture medium used in step (c), wherein the culture supernatant is subjected to conditions which allow the preparation of levan; or
(ii) comprised in a cell extract from the host organism cultivated in step (c), wherein the cell extract is subjected to conditions which allow the preparation of levan; or
(iii) comprised in a host organism or at the surface of the host organism cultivated in step (c), wherein the host organism is subjected to conditions which allow the preparation of levan; or
(iv) provided in a purified or immobilized form.

### BRIEF DESCRIPTION OF DRAWINGS

**Figure 1****.** Macroscopic image of different *G.* strains on yeast mannitol agar (A) and yeast sucrose agar (B). Photographic documentation of the plated strains *G. japonicus* LMG 1417 (1), *G. cerinus* LMG 1425 (2), *G. oxydans* LMG 1385 (3), *G. oxydans* DSM 2003 (4), *G. oxydans* DSM 3504 (5) and *G*. *oxydans* 621H (6) was performed after 24 hours of incubation at 28 °C. The incubation of *G. japonicus* LMG 1417 was additionally shown in time lapse (C).
**Figure 2****.** ¹³C-NMR spectra of EPS (1) produced by G. *japonicus* LMG 1417 and commercial levan (2) produced by *Erwinia herbicola* and obtained from Sigma-Aldrich (Blake et al. 1982). The measurements were performed using a Bruker Avance 300 DPX at a frequency of 75 MHz.
**Figure 3****.** FTIR spectra of EPS (black) produced by G. *japonicus* LMG 1417 and commercial levan (grey) produced by *Erwinia herbicola* (Blake et al. (1982) J. Bacteriol). The measurement was performed in absorption mode in a Bruker Tensor 27 FT-IR.
**Figure 4****.** Plasmid map of the overexpression plasmid pASK5_*levS1417.* Amp^{R}, ampicillin resistance cassette; ori, origin of replication.
**Figure 5****.** Silver stained SDS-PAGE (A), pH profile (B) and Michaelis-Menten kinetics (C) of the purified recombinant levansucrase from *G. japonicus* LMG 1417. The protein was purified by affinity chromatography after heterologous overproduction in *E. coli* DH5α. Marker of the SDS-PAGE: PageRuler Prestained Protein Ladder (ThermoFisher).
**Figure 6****.** Comparison of different specific levansucrase activities based on the protein database BRENDA (Jeske et al. (2019) Nucleic Acids Res., doi: 10.1093/nar/gky1048).
**Figure 7****.** Plasmid map of the modified broad host range vector pBBR1-p264-streplong (Zeiser et al. (2014) Appl. Microbiol. Biotechnol., doi: 10.1007/s00253-013-5016-5). Kan^{R}, kanamycin resistance cassette; rep, replication protein; mob; mob gene; p264, strong G.-specific promoter; MCS, multiple cloning site.
**Figure 8****.** Plasmid map of the overexpression plasmid pBBR1_p264_*levS1417*. Kan^{R}, kanamycin resistance cassette; rep, replication protein; mob, mob gene; p264, strong *Gluconobacter-*specific promoter; MCS, multiple cloning site.
**Figure 9****.** Schematic representation of the process solution for cell-free levan production. The total volume of the reaction was 10 mL. The reaction solution was incubated at 30 °C.
**Figure 10****.** Reaction kinetics of the cell-free levan production based on *G. japonicus* LMG 1417 (A). To simplify the visualization of the less concentrated fructooligosaccharides, the scaling of the Y-axis was adapted (B). All products with a concentration of more than 10 mM are shown.
**Figure 11****.** Reaction kinetics of cell-free levan production based on the genetically modified strain G. *japonicus* LMG 1417 pBBR1_p264_*levS1417* (A). To simplify the visualization of the less concentrated fructooligosaccharides, the scaling of the Y-axis was adapted (B). All products with a concentration of more than 10 mM are shown.
**Figure 12****.** Comparison of the yields of different processes for levan production. The levan concentration that can be generated per L reaction solution is shown on the left. Shown on the right is the maximum yield of levan that can be obtained with one liter of the corresponding culture.
**Figure 13****.** Plasmid map of the created overexpression plasmid *pASK5_levB2286.* Amp^{R}, ampicillin resistance cassette; ori, origin of replication.
**Figure 14****.** Comparison of the specific activities of different endolevanases. The quantification of the activities for the endolevanases from *A. chroococcum* DSM 2286, *Bacteroides thetaiotaomicron* DSM 2079 and *Bacillus licheniformis* DSM 13 was performed by HPLC.
**Figure 15****.** Product spectra of different endolevanases. The quantitative product analysis of the endolevanases from *A. chroococcum* DSM 2286 (A), *Bacteroides thetaiotaomicron* DSM 2079 (B) and *Bacillus licheniformis* DSM 13 (C) is presented.
**Figure 16****.** Plasmid map of the created overexpression plasmid pASK5_*levS1417_levB2286.* Amp^{R}, ampicillin resistance cassette; ori, origin of replication.
**Figure 17****.** Protein biochemical detection of recombinant levansucrase LevS1417 and endolevanase LevB2286 after heterologous production in *E. coli* and subsequent purification by streptactin affinity chromatography. The visualization was performed by western blotting (A) and silver staining (B).
**Figure 18****.** Schematic representation of the production of *E. coli* cell extract for the enzymatic production of levan-based FOS.
**Figure 19****.** Reaction kinetics of extract-based production of levan-type FOS from sucrose. Cell extract of the strain *E. coli* BL21 pASK5_*levS1417_levB2286* was used for the illustrated reaction.

### DETAILED DESCRIPTION OF THE INVENTION

The following detailed description refers to, by way of illustration, specific details and embodiments in which the invention may be practiced. These embodiments are described in sufficient detail to enable those skilled in the art to practice the invention. Other embodiments may be utilized and structural and logical changes may be made without departing from the scope of the invention. The various embodiments are not necessarily mutually exclusive, as some embodiments can be combined with one or more other embodiments to form new embodiments.

Unless otherwise defined, all technical and scientific terms used herein have the same meaning as commonly understood by one of ordinary skill in the art. The singular terms "a", "an" and "the" include plural referents unless context clearly indicates otherwise. Similarly, the word "or" is intended to include "and" unless the context clearly indicates otherwise. The term "comprises" means "includes". In case of conflict, the present specification, including explanations of terms, will control.

The terms "one or more" or "at least one", as interchangeably used herein, relate to at least 1, or at least 2, 3, 4, 5, 6, 7, 8, 9, 10, 11, 12, 13, 14, 15, 20, 25 or a plurality of species, e.g. nucleic acid molecules. In this connection, the term "plurality" means more than one, preferably 2 or more, such as up to 1000.

Numeric values specified without decimal places here refer to the full value specified with one decimal place, i.e. for example, 99 % means 99.0 %, unless otherwise defined.

The terms "about" or "approximately" or "approx.", in connection with a numerical value, refer to a variance of ±10 %, preferably ±5 %, more preferably ±2 %, more preferably ±1 %, more preferably ±0.1 %, and most preferably less than ±0.1 %, with respect to the given numerical value.

When an amount, a concentration or other values or parameters is/are expressed in form of a range, a preferable range, or a preferable upper limit value and a preferable lower limit value, it should be understood as that any ranges obtained by combining any upper limit or preferable value with any lower limit or preferable value are specifically disclosed, without considering whether the obtained ranges are clearly mentioned in the context.

"Essentially", for example in "essentially consists of" typically means that the fructooligosaccharides may comprise little amounts of further compounds of different formulas in addition to the mentioned formulas Fₘ and/or GFₙ. Preferably, the further compounds are comprised in amounts of less than 10 wt.-%, preferably less than 5 wt.-%, more preferably less than 1 wt.-%, more preferably less than 0.1 wt.-%, more preferably less than 0.01 wt.-%, more preferably less than 0.001 wt.-%, wherein most preferably the fructooligosaccharides consist of the compounds of the mentioned formulas, if not explicitly stated otherwise.

The terms "fructooligosaccharides" and "FOS", as used interchangeably herein, relate to oligomers of at least 3 monosaccharide units that are glycosidically linked to each other. Such oligomers typically comprise 2 or more and up to 100 fructose units. The oligomers may be linear or branched. The linkage between the individual units may be beta 2-6 glycosidic and/or beta 1-2 glycosidic. While it is not excluded that other monosaccharide units are present, the oligomers comprise at least 2 fructose units and are typically comprised of at least 65 mol.-% fructose units. If other monosaccharide units are present, these are for example glucose units and located on the terminus/termini of the fructose oligomer.

If not indicated otherwise, all monosaccharides referred to herein are in the D form. Accordingly, the term fructose means D-fructose and the term glucose means D-glucose. "Levan" relates to a polymer of β-2,6-glycosidically linked fructose units with the structural formula of a monomeric fructose unit of the levan backbone being shown below:

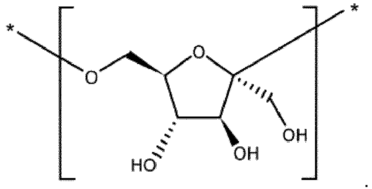

Levan may comprise a terminal glucose unit and may be branched by fructose units that are linked β-2,1-glycosidically.

The terms "preparation" "generation" and "production" or "preparing", "generating" or "producing" or "prepared", "generated" and "produced" are used synonymously.

The term "expression" includes every step (e.g. transcription, translation, protein folding) which is needed to produce the levansucrase and/or endolevanase in the host organism. The terms "levansucrase" and "endolevanase", as used herein, relate to functional enzymes that have the designated functionality of producing levan (from sucrose) and cleaving levan into FOS, respectively. The levansucrase is preferably an enzyme of EC 2.4.1.10, for example a prokaryotic or prokaryotic-derived enzyme, for example an enzyme originating from or derived from a bacterium of the genus *Gluconobacter, Zymomonas, Bacillus, Rahnella, Leuconostoc, Acetobacter* or *Erwinia,* in particular *Gluconobacter,* such as *Gluconobacter japonicus, Gluconobacter cerinus* or *Gluconobacter oxydans,* in particular *Gluconobacter japonicus.* The endolevanase is preferably an enzyme of EC 3.2.1.65, for example a prokaryotic or prokaryotic-derived enzyme, for example an enzyme originating from or derived from a bacterium of the genus *Azotobacter, Bacteroides* or *Bacillus,* in particular *Azotobacter,* such as *Azotobacter chroococcum.*

In a first aspect, the invention relates to a method for preparing fructooligosaccharides comprising or consisting of the following steps:
(a) optionally introducing at least one nucleic acid molecule comprising a first nucleotide sequence which encodes a levansucrase and/or a second nucleotide sequence which encodes an endolevanase into at least one host organism;
(b) providing
   (i) a host organism comprising a first nucleotide sequence which encodes a levansucrase and a second nucleotide sequence which encodes an endolevanase;
      or
   (ii) a first host organism comprising a first nucleotide sequence which encodes a levansucrase and a second host organism comprising the second nucleotide sequence which encodes an endolevanase;
(c) cultivating
   (i) the host organism comprising the first nucleotide sequence which encodes a levansucrase and the second nucleotide sequence which encodes an endolevanase under conditions which allow the expression of the first and the second nucleotide sequence;
      or
   (ii) the first host organism comprising the first nucleotide sequence which encodes a levansucrase and the second host organism comprising the second nucleotide sequence which encodes an endolevanase under conditions which allow the expression of the first and the second nucleotide sequence;
      and
(d) preparing fructooligosaccharides using the levansucrase and the endolevanase expressed in step (c), by subjecting the enzymes to conditions which allow the production of the fructooligosaccharides.

In a preferred embodiment, the at least one nucleic acid molecule is an expression vector.

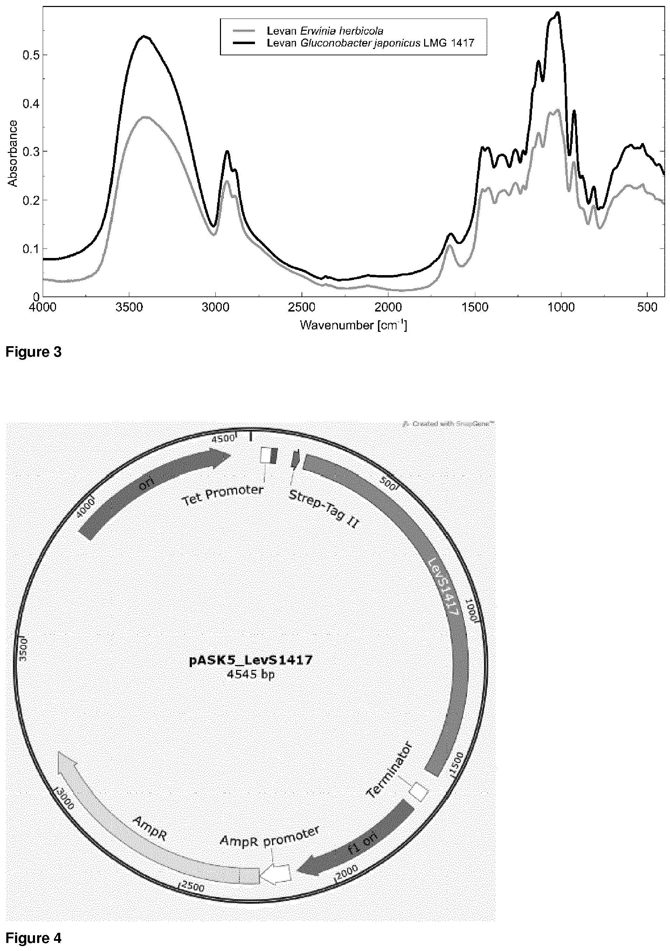

The terms "(expression) vector" and "(expression) plasmid" can be used synonymously and commonly refer to a circular DNA sequence which is used to transfer (foreign) genetic material into a target cell, if not stated otherwise. According to this invention, the expression vector preferably comprises at least one insert (sequence of interest), more preferably the first and/or the second nucleotide sequence as defined in the present invention. Most preferably, the purpose of the vector is to multiply or express the insert in the target cell, preferably to express the at least one insert, more preferably the first and/or the second nucleotide sequence, to obtain the at least one enzyme, in particular the levansucrase and/or the endolevanase which is encoded by the first nucleotide sequence and/or by the second nucleotide sequence. The person skilled in the art knows (expression) vectors which are suitable for the application described. A suitable expression vector, typically without an insert, is commercially available, e.g. from IBA GmbH. The at least one insert can be inserted later into the vector by typical (cloning) methods, which are known to the person skilled in the art. Examples of expression vectors comprising at least one insert and which can be used in the methods according to the invention, are illustrated in Figures 4, 8, 13 and 16.

In various embodiments, the first nucleotide sequence which encodes the levansucrase and/or the second nucleotide sequence which encodes the endolevanase according to the present invention is
(i) comprised in the expression vector; and/or
(ii) integrated in the genome, preferably in the chromosomal DNA, of the host organism.

In preferred embodiments, the first nucleotide sequence and/or the second nucleotide sequence according to the invention is integrated in the genome, preferably in the chromosomal DNA, of the host organism provided in step (b) of the method according to the invention. Most preferably, the first nucleotide sequence and the second nucleotide sequence are integrated in the genome, preferably in the chromosomal DNA, of the host organism provided in step (b) of the method according to the invention.

In a preferred embodiment of the method according to the invention,
in step (d) the levansucrase and the endolevanase are, individually or together,
(i) comprised in a culture supernatant from the culture medium used in step (c), wherein the culture supernatant is subjected to conditions which allow the preparation of said fructooligosaccharides; and/or
(ii) comprised in a cell extract from the at least one host organism cultivated in step (c), wherein the cell extract is subjected to conditions which allow the preparation of said fructooligosaccharides; and/or
(iii) comprised in a host organism or at the surface of the host organism cultivated in step (c), wherein the host organism is subjected to conditions which allow the preparation of said fructooligosaccharides; and/or
(iv) provided in a purified or immobilized form.

In various embodiments, the levansucrase and/or the endolevanase are released from the levansucrase and/or endolevanase producing host organism(s) during the cultivation process of step (c) and accumulate in the cell culture medium. The culture medium can be separated from the cells after step (c) (e.g. by centrifugation) to obtain the "culture supernatant" comprising the levansucrase and/or endolevanase. Preferably, the culture supernatant is cell-free. The culture supernatant can be used in step (d) of the method according to the invention by subjecting the "culture supernatant" to conditions which allow the preparation of FOS.

In another embodiment, the levansucrase and/or endolevanase producing host organism(s) are separated from the cell culture medium by centrifugation after step (c). The resulting cell pellet is in various embodiments subjected to cell disrupting methods to set free the contained cell components including the levansucrase and/or endolevanase. Suitable methods for cell disruption are known to the person skilled in the art (e.g. chemical or enzymatic lysis or mechanical methods, e.g. sonification). The composition obtained may be centrifuged and/or filtered and/or lyophilized before use in step (d) of the method according to the invention. The final composition is referred to as "cell extract" or "crude cell extract". Preferably, the cell extract is cell-free.

It is also possible, although not preferred, that the levansucrase and/or the endolevanase comprised in the cell extract of the host organism of step (c) are purified from the cell extract (e.g. by chromatography methods). Afterwards the purified enzymes can be used in step (d) of the method according to the invention to prepare said fructooligosaccharides. The purified enzymes can also be immobilized or lyophilized before using them in step (d). The person skilled in the art knows suitable immobilization techniques.

Furthermore, the levansucrase and the endolevanase do not have to be used in the same form in step (d) of the method according to the invention. For example, the levansucrase may be used as a cell extract and the endolevanase as a purified enzyme, or the levansucrase is used in its purified form and the endolevanase is used as a cell extract in step (d).

In a preferred embodiment, the method according to the invention comprises or consists of the following steps:
(a) optionally introducing one nucleic acid molecule comprising a first nucleotide sequence which encodes a levansucrase and a second nucleotide sequence which encodes an endolevanase into a host organism;
(b) providing a host organism comprising a first nucleotide sequence which encodes a levansucrase and a second nucleotide sequence which encodes an endolevanase;
(c) cultivating the host organism comprising the first nucleotide sequence which encodes a levansucrase and the second nucleotide sequence which encodes an endolevanase under conditions which allow the expression of the first and the second nucleotide sequence; and
(d) preparing said fructooligosaccharides using the levansucrase and the endolevanase expressed in step (c) and comprised in the cell extract from the host organism after step (c), and subjecting said cell extract to conditions which allow the preparation of said fructooligosaccharides.

In another preferred embodiment, the method according to the invention comprises or consists of the following steps:
(a) optionally introducing one nucleic acid molecule comprising a first nucleotide sequence which encodes a levansucrase and a second nucleotide sequence which encodes an endolevanase into a host organism;
(b) providing a host organism comprising a first nucleotide sequence which encodes a levansucrase and a second nucleotide sequence which encodes an endolevanase;
(c) cultivating the host organism comprising the first nucleotide sequence which encodes a levansucrase and the second nucleotide sequence which encodes an endolevanase under conditions which allow the expression of the first and the second nucleotide sequence; and
(d) preparing said fructooligosaccharides using the levansucrase and the endolevanase expressed in step (c) and comprised in the culture supernatant from the culture medium after step (c), and subjecting said culture supernatant to conditions which allow the preparation of said fructooligosaccharides.

In another preferred embodiment, the method for preparing fructooligosaccharides comprises or consists of the following steps:
(a) optionally introducing one nucleic acid molecule comprising a first nucleotide sequence which encodes a levansucrase and a second nucleotide sequence which encodes an endolevanase into one host organism, preferably the first nucleotide sequence and the second nucleotide sequence are comprised in one expression vector, which is introduced into the host organism;
(b) providing the host organism comprising a first nucleotide sequence which encodes a levansucrase and a second nucleotide sequence which encodes an endolevanase, preferably the first nucleotide sequence and the second nucleotide sequence are comprised in one expression vector;
(c) cultivating the host organism comprising the first nucleotide sequence which encodes a levansucrase and the second nucleotide sequence which encodes an endolevanase under conditions which allow the expression of the first and the second nucleotide sequence; and
(d) preparing said fructooligosaccharides using the levansucrase and the endolevanase expressed in step (c) and comprised in the host organism after step (c), and subjecting said host organism to conditions which allow the preparation of said fructooligosaccharides.

In another preferred embodiment, the method for preparing fructooligosaccharides comprises or consists of the following steps:
(a) optionally introducing one nucleic acid molecule comprising a first nucleotide sequence which encodes a levansucrase and/or a second nucleic acid molecule comprising a second nucleotide sequence which encodes an endolevanase into at least one host organism, wherein preferably the first nucleotide sequence is comprised in one expression vector and the second nucleotide sequence is comprised in a second expression vector, wherein the first and the second vectors are introduced into the same host organism or into different host organisms;
(b) providing
   (i) the host organism comprising a first nucleotide sequence which encodes a levansucrase and a second nucleotide sequence which encodes an endolevanase;
      or
   (ii) the first host organism comprising the first nucleotide sequence which encodes a levansucrase and the second host organism comprising the second nucleotide sequence which encodes an endolevanase;
(c) cultivating
   (i) the host organism comprising the first nucleotide sequence which encodes a levansucrase and the second nucleotide sequence which encodes an endolevanase under conditions which allow the expression of the first and the second nucleotide sequence;
      or
   (ii) the first host organism comprising the first nucleotide sequence which encodes a levansucrase and the second host organism comprising the second nucleotide sequence which encodes an endolevanase under conditions which allow the expression of the first and the second nucleotide sequence;
(d) preparing said fructooligosaccharides using the levansucrase and the endolevanase expressed in step (c) and comprised in the at least one host organism after step (c), and subjecting said at least one host organism to conditions which allow the preparation of said fructooligosaccharides.

"Comprised in the host organism" preferably means that the whole cells of the host organism, which comprise the levansucrase and/or the endolevanase, are used in step (d) without actively breaking or disrupting the cells. Preferably, the cells of the host organism are separated from the culture medium (e.g. by centrifugation) before they are used in step (d) of the method according to the invention.

In preferred embodiments, the at least one host organism is a prokaryotic or eukaryotic organism, preferably a bacterial or yeast organism, more preferably a bacterial organism, more preferably *Escherichia coli* or a *Gluconobacter* species, most preferably *Escherichia coli* BL21 or *Gluconobacter japonicus* LMG 1417.

The term "one nucleic acid molecule" or "at least one nucleic acid molecule" means one type or at least one type of nucleic acid molecule, but does not define the amount of the molecule. The term "at least one nucleic acid molecule comprising a first nucleotide sequence which encodes a levansucrase and/or a second nucleotide sequence which encodes an endolevanase" means that the first nucleotide sequence and the second nucleotide sequence can be combined in one nucleic acid molecule. However, it also means that the first nucleotide sequence can be comprised in one nucleic acid molecule and the second nucleotide sequence can be comprised in a second nucleic acid molecule.

Preferably, the first and the second nucleic acid molecules are expression vectors, also referred to as expression plasmids.

In various embodiments, the first nucleotide sequence and the second nucleotide sequence are comprised in one expression vector, which is introduced into the host organism.

In various embodiments, the first nucleotide sequence is comprised in one expression vector and the second nucleotide sequence is comprised in a second expression vector, wherein the first and the second vectors are introduced into the same host organism or into different host organisms.

Conditions, which allow the expression of the first and the second nucleotide sequence, are typical cultivation conditions, preferably used for *Gluconobacter* or *Escherichia coli* species, more preferably for *Escherichia coli* BL21 or *Gluconobacter japonicus* LMG 1417. Typically, these conditions allow the transcription of the first and/or second nucleotide sequence into the corresponding mRNA. Afterwards, it allows the translation of the formed mRNA into the respective corresponding amino acid chain and its folding to the corresponding enzyme. The production of the levansucrase and the endolevanase can take place in the same host organism or in different host organisms. For example, it is possible, that the levansucrase is produced in *Gluconobacter japonicus* and the endolevanase is producted in *Escherichia coli* BL21. In this case, the cultivation conditions for the two host organisms can be different in step (c) to allow the production of the single enzymes.

In preferred embodiments, the levansucrase and the endolevanase are produced in the same host organism, preferably in *Gluconobacter japonicus* LMG 1417 or *Escherichia coli* BL21.

In various embodiments, the host organism is selected such that either the levansucrase or the endolevanase or both are heterologous to the host organism, i.e. the host organism does not naturally express these enzymes. In such embodiments, the host organism is genetically engineered to express the heterologous enzyme(s).

In various embodiments, the cultivation of step (c) of the method according to the invention is carried out at 20 to 37 °C, preferably at 25 to 35 °C, more preferably at 28 °C or 30 °C. In various embodiments, the cultivation is carried out for up to 72 hours, preferably for up to 48 hours, more preferably for up to 24 hours.

In a preferred embodiment, in step (a), at least one nucleic acid molecule, preferably one nucleic acid molecule, comprising a first nucleotide sequence which encodes a levansucrase and a second nucleotide sequence which encodes an endolevanase is introduced into one host organism. Preferably, the host organism is a *Gluconobacter* or *Escherichia coli* species, more preferably *Escherichia coli* BL21.
Preferably, in step (b) one host organism comprising the first nucleotide sequence which encodes a levansucrase and the second nucleotide sequence which encodes an endolevanase is provided. Further preferred, the host organism, preferably *Escherichia coli* BL21, is cultivated in step (c) under conditions which allow the expression of the first and the second nucleotide sequence (the term "expression" typically comprises every step that is needed to produce the levansucrase and endolevanase in the host organism). Then in step (d), the host organism of step (c), which comprises the levansucrase and the endolevanase, is subjected to condition which allow the preparation of fructooligosaccharides. That means that the whole cells of the host organism comprising the levansucrase and the endolevanase are subjected to conditions which allow the production of fructooligosaccharides.

In another preferred embodiment, a cell extract is prepared from the host organism after step (c). The cell extract comprises the levansucrase and the endolevanase, which were produced in the host organism in step (c). This cell extract is used in step (d) to obtain the fructooligosaccharides by subjecting the cell extract to conditions which allow the preparation of said fructooligosaccharides.

In another embodiment, the levansucrase and the endolevanase can be purified from the cell extract of the host organism. The purified enzymes or alternatively the purified immobilized enzymes can be subjected to conditions in step (d) which allow the preparation of said fructooligosaccharides.

In a preferred embodiment, the culture supernatant comprising the levansucrase and the endolevanase is used in step (d) of the method according to the invention to obtain the fructooligosaccharides by subjecting the culture supernatant to conditions which allow the preparation of said FOS.

In another preferred embodiment, the host organism is a *Gluconobacter* species, preferably *Gluconobacter japonicas,* more preferably *Gluconobacter japonicus* LMG 1417, which comprises the first nucleotide sequence which encodes for the levansucrase naturally in its genome. The wildtype host organism can be used to produce the levansucrase. It is not necessary to introduce a nucleic acid molecule comprising the first nucleotide sequence which encodes the levansucrase into the host organism. The nucleic acid molecule comprising the second nucleotide sequence which encodes the endolevanase can be introduced into the same host organism or into another host organism before starting step (c).

However, it is preferred that the host organism, preferably a *Gluconobacter* species, more preferably *Gluconobacter japonicus,* most preferably *Gluconobacter japonicus* LMG 1417, comprises the first nucleotide sequence which encodes for the levansucrase in its genome, preferably in its chromosomal DNA and, additionally, comprises at least one nucleic acid molecule, preferably an expression vector, comprising the first nucleotide sequence which encodes the levansucrase. In this case, it is expected that the levansucrase production will be increased in the cell in comparison to the wildtype host organism, which comprises the first nucleotide sequence only in its genome. In addition, the host organism may comprise the second nucleotide sequence, which encodes the endolevanases, in the afore-mentioned nucleic acid molecule or in a second nucleic acid molecule.

In another preferred embodiment, the host organism comprises the first nucleotide sequence which encodes the levansucrase and the second nucleotide sequence which encodes the endolevanase in its genome, preferably in its chromosomal DNA. This host organism is provided in step (b) of the method according to the invention. Typically, the sequences were previously integrated in the host's chromosomal DNA by biotechnological methods, which are known to the person skilled in the art. Preferably, the host organism is a *Gluconobacter* or *Escherichia coli* species, preferably an *Escherichia coli* species.

In another preferred embodiment, the host organism comprises the first nucleotide sequence which encodes the levansucrase in its genome, preferably in its chromosomal DNA. In various embodiments, the host organism comprises the first nucleotide sequence which encodes the levansucrase naturally in its genome, preferably in its chromosomal DNA. The second nucleotide sequence which encodes the endolevanase is comprised in an expression vector in the host organism. This host organism is provided in step (b) of the method according to the invention.

In another embodiment, the host organism comprises the second nucleotide sequence which encodes the endolevanase in its genome. The first nucleotide sequence which encodes the levansucrase is comprised in an expression vector in the host organism. This host organism is provided in step (b) of the method according to the invention.
In a preferred embodiment, the levansucrase can be used in step (d)
(i) in purified or immobilized form; or
(ii) comprised in a cell extract from the host organism; or
(iii) comprised in the host organism or at the surface of the host organism; or
(iv) comprised in the culture supernatant.

In a preferred embodiment, the endolevanase can be used in step (d)
(i) in purified or immobilized form; or
(ii) comprised in a cell extract from the host organism; or
(iii) comprised in the host organism or at the surface of the host organism; or
(iv) comprised in the culture supernatant.

Most preferred, the levansucrase used in step (d) is comprised in a cell extract from the host organism. The cell extract may comprise only the levansucrase, or the levansucrase and the endolevanase.

Most preferred, the endolevanase used in step (d) is comprised in a cell extract from the host organism. The cell extract may comprise only the endolevanase, or the levansucrase and the endolevanase.

Further preferred, the levansucrase used in step (d) is comprised in a culture supernatant from the culture medium after step (c). The culture supernatant may comprise only the levansucrase, or the levansucrase and the endolevanase.

Further preferred, the endolevanase used in step (d) is comprised in a culture supernatant from the culture medium after step (c). The culture supernatant may comprise only the endolevanase, or the levansucrase and the endolevanase.

In the case the cell extract or the culture supernatant comprises only one of the enzymes, the second enzyme has to be added in step (d) of the method for preparing FOS according to the invention, as well, preferably in purified or immobilized form or comprised in a (second) cell extract, in a (second) culture supernatant or comprised in the host organism or at the surface of the host organism.

It is possible, that the two enzymes are used (or applied or added) in step (d) simultaneously or sequentially.

In one embodiment, first the levansucrase is added in step (d) to produce levan from a substrate, preferably from the substrate sucrose. After levan has been formed, the endolevanase is added to hydrolyze the levan to the fructooligosaccharides. In this case, the enzymes are used sequentially.

In another embodiment, the levansucrase and the endolevanase are used (or applied or added) simultaneously in step (d) to form the suitable fructooligosaccharides.

In both embodiments, the two enzymes can be used (or applied or added) in purified form or comprised in a cell extract from the host organisms, in a culture supernatant from the culture medium or comprised in the host organism or at the surface of the host organism.

Suitable methods to use (or apply or add) the levansucrase and the endolevanase in step (d) are known to the person skilled in the art. Some of these methods have already been described above and may be comprised in at least one further step, which is comprised in the method according to the invention between step (c) and step (d).

In various embodiments, the method according to the invention comprises after step (d) a further step (e) for purifying the fructooligosaccharides obtained in step (d).

Preferably, step (e) is a chromatography step or a filtration step, without being limited to these methods. The person skilled in the art knows which techniques are most appropriate.

In preferred embodiments, the conditions which allow the preparation of the fructooligosaccharides of step (d) comprise providing sucrose to the levansucrase, or to the levansucrase and endolevanase.

Preferably, the sucrose is converted to the fructooligosaccharides obtained in step (d), wherein the conversion is catalyzed by the levansucrase and the endolevanase.

Preferably, the levansucrase hydrolyzes the sucrose to form fructan-polymers, also referred to as levan, preferably, the fructan-polymers comprise up to 100.000 fructose units,
and then the endolevanase hydrolyses the fructan-polymers to generate the fructooligosaccharides obtained in step (d).

In various embodiments, a mixture of (short-chain) fructooligosaccharides of different lengths is obtained in step (d) of the method for preparing fructooligosaccharides according to the invention.

In a preferred embodiment, the (short-chain) fructooligosaccharides obtained in step (d) comprise, essentially consist of or consist of compounds of the formulas Fₘ and/or GFₙ, wherein
F is a monomeric fructose unit, preferably D-fructose unit;
G is a monomeric glucose unit, preferably D-glucose unit;
m is ≥3, preferably 3 to 20, more preferably 3 to 15, more preferably 3 to 13, most preferably 3 to 10;
n is ≥2, preferably 2 to 19, more preferably 2 to 14, more preferably 2 to 12, most preferably 2 to 9;
m and/or n are the same or different in the individual fructooligosaccharides obtained in step (d); and the fructose units are covalently coupled to each other by β-(2→-6) linkages and may further comprise β-(2→-1) branching..

Preferably, the mixture comprises, essentially consists of or consists of compounds of the formulas Fₘ and GFₙ of different lengths as defined above.

In various embodiments, the mixture of (short-chain) fructooliosaccharides of different lengths further comprises compounds of the formula Fₘ, wherein m is 1 and/or 2 (free fructose and/or levanbiose).

In various embodiments, the amount of compounds of the formula Fₘ, wherein m is 1 and/or 2, preferably free fructose and/or levanbiose, is reduced in the fructooligosaccharides obtained in step (d) of the method for preparing fructooligosaccharides according to the invention (catalyzed by the levansucrase and endolevanase as defined according to the invention) or in the levan obtained in step (d) of the method for preparing levan according to the invention (catalyzed by the levansucrase as defined according to the invention), in comparison to methods which use different enzymes or enzyme combinations. Preferably, the amount of compounds of the formula Fₘ, wherein m is 1 and/or 2 is less than 12 %, preferably less than 8 % based on the amount of fructose units which are comprised in the sucrose which is added in step (d) of the methods according to the invention.

Fructooligosaccharides of the formula Fₘ as obtainable by the method according to the invention, typically only consist of monomeric fructose units which are linked to each other, preferably by beta-2,6-glycosidic bonds. A monomeric fructose unit is illustrated in the following formula:

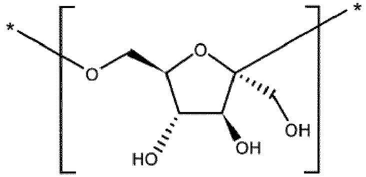

Fructooligosaccharides of the formula GFₘ as obtainable by the method according to the invention, typically consist of one monomeric glucose unit which is linked to a terminal fructose unit of a fructose chain, wherein the fructose units of the fructose chain are preferably linked to each other by beta-2,6-glycosidic bonds.

Furthermore, the fructooligosaccharides obtained in step (d) are preferably of the levan-type.

The term "levan-type" or "levan-based" typically comprises oligo- and polysaccharides, which contain two or more fructose units, wherein the single fructose units are (mainly) linked to each other by beta-2,6-glycosidic bonds, if not explicitly stated otherwise.

Additionally, the levansucrase preferably converts sucrose to fructan-polymers, preferably with up to 100.000 fructose units, which are linked to each other by beta-2,6-glycosidic bonds. This intermediate can be hydrolyzed by the endolevanase to form smaller fructooligosaccharide compounds of the formula Fₘ and/or GFₙ, wherein m is ≥3, preferably 3 to 20, more preferably 3 to 15, more preferably 3 to 13, most preferably 3 to 10; and n is ≥2, preferably 2 to 19, more preferably 2 to 14, more preferably 2 to 12, most preferably 2 to 9; wherein m and/or n may be different in the respective fructooligosaccharides obtained in step (d).

In a preferred embodiment, the fructooligosaccharides obtainable by the method according to the invention have a molecular weight of up to 3258 g mol⁻¹, more preferably up to 2448 g mol⁻¹, and most preferably up to 1638 g mol⁻¹.

In another preferred embodiment of the method for preparing fructooligosaccharides according to the invention, at least 30 % or 40 % or 50 % or 60 % or 70 % or 75 % or 80 % or 85 % or 86 % or 87 % or 88 % or 89 % or 90 % or 91 % or 92 % or 93 % or 94 % or 95 % or 96 % or 97 % or 98 % or 99 % or 99,9 % of the sucrose is converted to fructooligosaccharides, preferably to fructooligosaccharides of the formulas Fₘ and/or GFₙ, wherein F, G, m and n are as defined above, based on the sucrose concentration and the amount of levansucrase and endolevanase which is added in step (d) of the method according to the invention. Preferably, the sucrose is converted within 600 hours, more preferably within 500 hours, more preferably within 490 hours, more preferably within 480 hours, more preferably within 400 hours, more preferably within 300 hours, more preferably within 260 hours, more preferably within 200 hours, more preferably within 100 hours, more preferably within 60 hours, more preferably within 50 hours, more preferably within 49 hours, more preferably within 48 hours, more preferably within 40 hours, more preferably within 30 hours, more preferably within 26 hours, more preferably within 20 hours, more preferably within 15 hours, more preferably within 10 hours, more preferably within 5 hours, based on the sucrose concentration and the amount of levansucrase and endolevanase which is added in step (d) of the method according to the invention. In various embodiments, the time of conversion from sucrose to fructooligosaccharides according to the present invention can be reduced by adding increased amounts of levansucrase and endolevanase in step (d) of the method according to the invention. In preferred embodiments, the levansucrase and the endolevanase are comprised in culture supernatant or cell extract or whole cells of the host organism. Thus, increased amounts of culture supernatant or cell extract or whole cells comprising the levansucrase and the endolevanase result in a faster conversion from sucrose to FOS in step (d) of the method according to the invention. In various embodiments, sucrose is added in step (d) in amounts of up to 5 mol L⁻¹, up to 3 mol L⁻¹, up to 2.5 mol L⁻¹, up to 2 mol L⁻¹, up to 1.5 mol L⁻¹, up to 1 mol L⁻¹, up to 0.5 mol L⁻¹, up to 0.25 mol L⁻¹, up to 0.2 mol L⁻¹, up to 0.15 mol L⁻¹, up to 0.1 mol L⁻¹, up to 0.05 mol L⁻¹ or up to 0.01 mol L⁻¹. In various embodiments, sucrose is added in step (d) in amounts of 5 mol L⁻¹, 3 mol L⁻¹, 2.5 mol L⁻¹, 2 mol L⁻¹, 1.5 mol L⁻¹, 1 mol L⁻¹, 0.5 mol L⁻¹, 0.25 mol L⁻¹, 0.2 mol L⁻¹, 0.15 mol L⁻¹, 0.1 mol L⁻¹, 0.05 mol L⁻¹ or 0.01 mol L⁻¹. In various embodiments, the conversion of sucrose to FOS is carried out at 20 to 37 °C, preferably at 25 to 35 °C, more preferably at 28 °C or 30 °C.

In various embodiment, the levansucrase comprises or consists of
(i) an amino acid sequence set forth in SEQ ID Nos. 1 or 2; or
(ii) an amino acid sequence, which has at least 50 %, at least 60 %, at least 70 %, at least 75 %, at least 80 %, at least 85 %, at least 90 %, at least 91 %, at least 92 %, at least 93 %, at least 94 %, at least 95 %, at least 96 %, at least 97 %, at least 98 % or at least 99 % sequence identity with the amino acid sequence set forth in SEQ ID Nos. 1 or 2 over the full length of the sequence.

In a preferred embodiment, the levansucrase comprises or consists of the amino acid sequence set forth in SEQ ID NO:2 or a fragment thereof lacking the N-terminal methionine (M) residue. Further suitable fragments of the above-described levansucrases include, but are not limited to those that retain at least 75 % of the activity of the full length sequence but lack one or more N-terminal and/or C-terminal amino acids.

In a preferred embodiment, the first nucleotide sequence, which encodes the levansucrase, is originated from *Gluconobacter japonicus* LMG 1417.

In a preferred embodiment, the first nucleotide sequence, which encodes the levansucrase, comprises or consists of a nucleotide sequence
(i) set forth in SEQ ID Nos. 3 or 4; or
(ii) which has at least 50 %, at least 60 %, at least 70 %, at least 75 %, at least 80 %, at least 85 %, at least 90 %, at least 91 %, at least 92 %, at least 93 %, at least 94 %, at least 95 %, at least 96 %, at least 97 %, at least 98 % or at least 99 % sequence identity with the nucleotide sequence set forth in SEQ ID Nos. 3 or 4 over the full length of the sequence.

In various embodiments, these nucleotide sequences encode levansucrases that comprise or consist of the amino acid sequence set forth in SEQ ID Nos. 1 or 2 or an amino acid sequence which has at least 60 %, 70 %, 75 %, 80 %, 85 %, 90 %, 91 %, 92 %, 93 %, 94 %, 95 %, 96 %, 97 %, 98 % or 99 % sequence identity with the amino acid sequence set forth in SEQ ID Nos. 1 or 2 over the full length of the sequence or fragments thereof, as defined above.

In one embodiment, the first nucleotide sequence, which encodes the levansucrase, comprises or consists of a nucleotide sequence set forth in SEQ ID NO:3.

In another preferred embodiment, the first nucleotide sequence, which encodes the levansucrase, comprises or consists of a nucleotide sequence set forth in SEQ ID NO:4.

In various embodiments, the endolevanase comprises or consists of
(i) an amino acid sequence set forth in SEQ ID Nos. 5 or 6; or
(ii) an amino acid sequence, which has at least 50 %, at least 60 %, at least 70 %, at least 75 %, at least 80 %, at least 85 %, at least 90 %, at least 91 %, at least 92 %, at least 93 %, at least 94 %, at least 95 %, at least 96 %, at least 97 %, at least 98 % or at least 99 % sequence identity with the amino acid sequence set forth in SEQ ID Nos. 5 or 6 over the full length of the sequence.

In a preferred embodiment, the endolevanase comprises or consists of the amino acid sequence set forth in SEQ ID NO:6 or a fragment thereof that lacks the first 35 N-terminal amino acids and starts with A36. Further suitable fragments of the above-described levansucrases include, but are not limited to those that retain at least 75 % of the activity of the full length sequence but lack one or more N-terminal and/or C-terminal amino acids. Preferred fragments include those of SEQ ID NO:6 that lack one or more, such as 2, 3, 4, 5, 6, 7, 8, 9, 10, 11, 12 ,13, 14, 15, 16, 17, 18, 19, 20, 21, 22, 23, 24, 25, 26, 27, 28, 29, 30, 31, 32, 33, 34 or up to 35 amino acids from the N-terminus.

In a preferred embodiment, the second nucleotide sequence, which encodes the endolevanase, is originated from *Azotobacter chroococcum* DSM 2286.

In a preferred embodiment, the second nucleotide sequence, which encodes the endolevanase, comprises or consists of a nucleotide sequence
(i) set forth in SEQ ID Nos. 7 or 8; or
(ii) which has at least 50 %, at least 60 %, at least 70 %, at least 75 %, at least 80 %, at least 85 %, at least 90 %, at least 91 %, at least 92 %, at least 93 %, at least 94 %, at least 95 %, at least 96 %, at least 97 %, at least 98 % or at least 99 % sequence identity with the nucleotide sequence set forth in SEQ ID Nos. 7 or 8 over the full length of the sequence.

In various embodiments, these nucleotide sequences encode endolevanases that comprise or consist of the amino acid sequence set forth in SEQ ID Nos. 5 or 6 or an amino acid sequence which has at least 60 %, 70 %, 75 %, 80 %, 85 %, 90 %, 91 %, 92 %, 93 %, 94 %, 95 %, 96 %, 97 %, 98 % or 99 % sequence identity with the amino acid sequence set forth in SEQ ID Nos. 5 or 6 over the full length of the sequence or fragments thereof, as defined above.

Most preferably, the second nucleotide sequence which encodes the endolevanase comprises or consists of a nucleotide sequence set forth in SEQ ID NO:7 or 8.

The term "sequence identity" as used herein typically refers to amino acid sequences that share identical amino acids at corresponding positions or nucleotide sequences sharing identical nucleotides at corresponding positions, if not explicitly stated otherwise. Amino acid sequences with a sequence identity of less than 100 % typically relate to amino acid sequences which have one or more amino acids added, deleted, substituted or otherwise modified in comparison to another amino acid sequence that serves as a reference. In various embodiments, the given sequence identity refers to the sequence identity over the entire length of the reference sequence. This means that if a reference sequence is 100 amino acids in length, any query sequence that needs to have a sequence identity of, for example, 70 %, needs to have at least 70 identical amino acids in corresponding positions over the 100 amino acid long stretch of the reference sequence when both are properly aligned. These 70 identical amino acids may be contiguous but do not need to be contiguous. This also means that the query sequence is at least 70 amino acids in length. The remaining 30 amino acids may differ between both sequences. A similar definition of "sequence identity" applies to nucleotide sequences. Here the identity refers to identical nucleotides in corresponding positions.

The determination of percent identity described herein between two amino acid or nucleotide sequences can be accomplished using a mathematical algorithm. For example, a mathematical algorithm useful for comparing two sequences is the algorithm of Karlin and Altschul (1990, Proc. Natl. Acad. Sci. USA 87:2264-2268), modified as in Karlin and Altschul (1993, Proc. Natl. Acad. Sci. USA 90:5873-5877). This algorithm is incorporated into the BLASTN and BLASTX programs and can be accessed, for example, at the National Center for Biotechnology Information (NCBI) world wide web site having the universal resource locator "www.ncbi.nlm.nih.gov/BLAST". Blast nucleotide searches can be performed with BLASTN program, whereas BLAST protein searches can be performed with BLASTX program or the NCBI "blastp" program. Another algorithm available in the art is the FASTA algorithm. Sequence comparisons (alignments), in particular multiple sequence comparisons, can be generated using computer programs. Commonly used are for example the Clustal series (See, e.g. ,Chenna et al. (2003): Multiple sequence alignment with the Clustal series of programs. Nucleic Acid Research 31, 3497-3500), T-Coffee (See, e.g., Notredame et al. (2000): T-Coffee: A novel method for multiple sequence alignments. J. Mol. Biol. 302, 205-217) or programs based thereon or the respective algorithms. Further possible are sequence comparisons (alignments) with the computer program Vector NTI® Suite 10.3 (Invitrogen Corporation, 1600 Faraday Avenue, Carlsbad, CA, USA) with the pre-set standard parameters, the AlignX-module of which is based on ClustalW. If not explicitly defined otherwise, sequence identity is determined using the BLAST algorithm.

In preferred embodiments, the levansucrase has a specific activity of at least 1000 U/mg, preferably of at least 2000 U/mg, more preferably of at least 2500 U/mg, most preferably of at least 3000 U/mg, measured based on Michaelis-Menten kinetics. Preferably, the levansucrase is from *Gluconobacter japonicus* LMG 1417, produced in *Escherichia coli*, and purified by affinity chromatography. More preferably, the specific activity is measured at 20 to 37 °C, preferably at 25 to 35 °C, most preferably at approx. 30 °C. Additionally, the pH is preferably between 5.0 and 6.0, more preferably between 5.2 and 6.8, most preferably the pH is approx. 5.4.

In preferred embodiments, the endolevanase has a specific activity of at least 500 U/mg, preferably of at least 550 U/mg, more preferably of at least 750 U/mg, more preferably of at least 950 U/mg, most preferably of at least 1000 U/mg, measured based on Michaelis-Menten kinetics. Preferably, the endolevanase is from *Azotobacter chroococcum* DSM 2286, produced in *Escherichia coli*, and purified by affinity chromatography. More preferably, the specific activity is measured at 20 to 37 °C, preferably at 25 to 35 °C, most preferably at approx. 30 °C. Additionally, the pH is preferably between 5.5 and 6.5, more preferably between 5.7 and 6.3, most preferably the pH is approx. 6.0.

In another aspect, the invention relates to a method for preparing levan comprising or consisting of the following steps:
(a) optionally introducing at least one nucleic acid molecule comprising a first nucleotide sequence which encodes a levansucrase into a host organism;
(b) providing a host organism comprising a first nucleotide sequence which encodes a levansucrase;
(c) cultivating the host organism under conditions which allow the expression of the first nucleotide sequence;and
(d) preparing levan using the levansucrase expressed in step (c), and subjecting it to conditions which allow the preparation of levan.

Preferably, in step (d) of the method for preparing levan according to the invention, the levansucrase is
(i) comprised in a culture supernatant of the host organism cultivated in step (c), wherein the culture supernatant is subjected to conditions which allow the preparation of levan; or
(ii) comprised in a cell extract from the host organism cultivated in step (c), wherein the cell extract is subjected to conditions which allow the preparation of levan; or
(iii) comprised in a host organism or at the surface of the host organism cultivated in step (c), wherein the host organism is subjected to conditions which allow the preparation of levan; or
(iv) provided in a purified or immobilized form.

In a preferred embodiment, the first nucleotide sequence, which encodes a levansucrase, originates from a *Gluconobacter* species. In various embodiments, it may comprise or consist of the nucleotide sequences encoding levansucrase disclosed herein. In various embodiments, the host organism may be a *Gluconobacter* species as well, preferably *Gluconobacter japonicus* such as *Gluconobacter japonicus* LMG 1417. This host organism comprises the first nucleotide sequence in its genome, preferably in its chromosomal DNA. However, in such embodiments, it may be preferred that a nucleic acid molecule comprising the first nucleotide sequence, which encodes the levansucrase, is additionally introduced in the host organism to increase expression of the levansucrase. In such embodiments, the host organism would still be genetically engineered although the introduced coding sequence is homologous. Preferably, the nucleic acid molecule is an expression vector/plasmid. Such an expression vector/plasmid may comprise sequence elements, for example regulatory elements, such as promotors and the like, that are heterologous to the host organism.

In various embodiments, the cultivation process of step (c) of the method for preparing levan according to the invention is carried out at 20 to 37 °C, preferably at 25 to 35 °C, more preferably at 28 °C or 30 °C. In various embodiments, the cultivation is carried out for up to 72 hours, preferably for up to 48 hours, more preferably for up to 24 hours.

Preferably, the culture supernatant or the cell extract comprising the levansucrase is added in step (d) of the method according to the invention to form levan from sucrose.

More preferably, the levansucrase of step (d) is subjected to sucrose to catalyze the conversion of sucrose to fructan-polymers. Levan may comprise up to 100.000 fructose units which are (mainly) linked to each other by beta-2,6-glycosidic bonds.

In a preferred embodiment of the method for preparing levan according to the invention, at least 30 % or 40 % or 50 % or 60 % or 70 % or 75 % or 80 % or 85 % or 86 % or 87 % or 88 % or 89 % or 90 % or 91 % or 92 % or 93 % or 94 % or 95 % or 96 % or 97 % or 98 % or 99 % or 99,9 % of the sucrose is converted to levan, based on the sucrose concentration and the amount of levansucrase which is added in step (d) of the method according to the invention. Preferably, the sucrose is converted within 600 hours, more preferably within 500 hours, more preferably within 490 hours, more preferably within 480 hours, more preferably within 400 hours, more preferably within 300 hours, more preferably within 260 hours, more preferably within 200 hours, more preferably within 100 hours, more preferably within 60 hours, more preferably within 50 hours, more preferably within 49 hours, more preferably within 48 hours, more preferably within 40 hours, more preferably within 30 hours, more preferably within 26 hours, more preferably within 20 hours, more preferably within 15 hours, more preferably within 10 hours, more preferably within 5 hours, based on the sucrose concentration and the amount of levansucrase which is added in step (d) of the method according to the invention. In various embodiments, the time of conversion from sucrose to levan can be reduced by adding increased amounts of levansucrase in step (d) of the method according to the invention. In preferred embodiments, the levansucrase is comprised in culture supernatant or cell extract or whole cells of the host organism. Thus, increased amounts of culture supernatant or cell extract or whole cells comprising the levansucrase and the endolevanase result in a faster conversion from sucrose to levan in step (d) of the method according to the invention. For example, the conversion time can be reduced to approx. one tenth if the enzyme is added in a 10-fold concentration. In various embodiments, sucrose is added in step (d) in amounts of up to 5 mol L⁻¹ or up to 3 mol L⁻¹ or up to 2.5 mol L⁻¹ or up to 2 mol L⁻¹ or up to 1.5 mol L⁻¹ or up to 1 mol L⁻¹ or up to 0.5 mol L⁻¹ or up to 0.25 mol L⁻¹ or up to 0.2 mol L⁻¹ or up to 0.15 mol L⁻¹ or up to 0.1 mol L⁻¹ or up to 0.05 mol L⁻¹ or up to 0.01 mol L⁻¹. In various embodiments, sucrose is added in step (d) in amounts of 5 mol L⁻¹ or 3 mol L⁻¹ or 2.5 mol L⁻¹ or 2 mol L⁻¹ or 1.5 mol L⁻¹ or 1 mol L⁻¹ or 0.5 mol L⁻¹ or 0.25 mol L⁻¹ or 0.2 mol L⁻¹ or 0.15 mol L⁻¹ or 0.1 mol L⁻¹ or 0.05 mol L⁻¹ or 0.01 mol L⁻¹. In various embodiments, the conversion from sucrose to levan is carried out at 20 to 37 °C, preferably at 25 to 35 °C, more preferably at 28 °C or 30 °C.

It is possible that, besides levan, further fructooligosaccharides are produced from sucrose, which are preferably compounds of the formula Fₘ and/or GFₙ, wherein F, G, m and n are as defined above. These further fructooligosaccharides typically result from the levansucrase catalysis as well.

In another aspect, the invention relates to a method for preparing fructoligosaccharides from levan comprising or consisting of the following steps:
(a) optionally introducing at least one nucleic acid molecule comprising a second nucleotide sequence which encodes an endolevanase into a host organism;
(b) providing a host organism comprising a second nucleotide sequence which encodes an endolevanase;
(c) cultivating the host organism under conditions which allow the expression of the second nucleotide sequence;and
(d) preparing fructooligosaccharides from levan using the endolevanase expressed in step (c), and subjecting it to conditions which allow the production of fructooligosaccharides.

Preferably, in step (d) of the method for preparing FOS according to the invention, the endolevanase is
(i) comprised in a culture supernatant of the host organism cultivated in step (c), wherein the culture supernatant is subjected to conditions which allow the preparation of FOS; or
(ii) comprised in a cell extract from the host organism cultivated in step (c), wherein the cell extract is subjected to conditions which allow the preparation of FOS; or
(iii) comprised in a host organism or at the surface of the host organism cultivated in step (c), wherein the host organism is subjected to conditions which allow the preparation of FOS; or
(iv) provided in a purified or immobilized form.

In a preferred embodiment, the second nucleotide sequence, which encodes an endolevanase, originates from an *Azotobacter* species. In various embodiments, it may comprise or consist of the nucleotide sequences encoding endolevanase disclosed herein. In various embodiments, the host organism may be *Azotobacter chroococcum* DSM 2286. This host organism comprises the second nucleotide sequence in its genome, preferably in its chromosomal DNA. However, in such embodiments, it may be preferred that a nucleic acid molecule comprising the second nucleotide sequence, which encodes the endolevanase, is additionally introduced in the host organism to increase expression of the endolevanase. In such embodiments, the host organism would still be genetically engineered although the introduced coding sequence is homologous. Preferably, the nucleic acid molecule is an expression vector/plasmid. Such an expression vector/plasmid may comprise sequence elements, for example regulatory elements, such as promotors and the like, that are heterologous to the host organism.

In another aspect, the invention relates to fructooligosaccharides (FOS) obtainable by the method for preparing fructooligosaccharides according to the invention.

In still another aspect, the invention relates to at least one expression vector comprising a first nucleotide sequence which encodes a levansucrase and/or a second nucleotide sequence which encodes an endolevanase;
wherein the first nucleotide sequence which encodes the levansucrase comprises or consists of a nucleotide sequence
(i) set forth in SEQ ID Nos. 3 or 4; or
(ii) which has at least 70 %, 75 %, 80 %, 85 %, 90 %, 91 %, 92 %, 93 %, 94 %, 95 %, 96 %, 97 %, 98 % or 99 % sequence identity with the nucleotide sequence set forth in SEQ ID Nos. 3 or 4 over the full length of the sequence; and/or
wherein the second nucleotide sequence which encodes the endolevanase comprises or consists of a nucleotide sequence
(i) set forth in SEQ ID Nos. 7 or 8; or
(ii) which has at least 70 %, 75 %, 80 %, 85 %, 90 %, 91 %, 92 %, 93 %, 94 %, 95 %, 96 %, 97 %, 98 % or 99 % sequence identity with the nucleotide sequence set forth in SEQ ID Nos. 7 or 8 over the full length of the sequence.

In a still further aspect, the invention relates to a genetically modified host organism comprising
(i) the expression vector as defined in the third aspect; and/or
(ii) the first nucleotide sequence which encodes the levansucrase and/or the second nucleotide sequence which encodes the endolevanase according to the present invention in its genome, preferably in its chromosomal DNA; and/or
(iii) the levansucrase and/or the endolevanase according to the present invention.

The term "genetically modified host organism" typically comprises organisms which comprise foreign DNA, preferably at least one nucleic acid molecule according to the invention (e.g. an expression vector), and/or which are modified in their genome sequence, if not explicitly stated otherwise. The host organism is, in various embodiments, selected such that at least one of the introduced nucleotide sequences is heterologous relative to the host organism. For example, if the host organism is *Gluconobacter japonicus* LMG 1417, the introduced sequences may comprise the sequence encoding the endolevanase derived from *Azotobacter chroococcum* DSM 2286 and vice versa.

Preferably, the term "host organism" as used in steps (b) and (c) (and partly in step (d)) of the methods according to the invention refers to the "genetically modified host organism" as described in aspect four.

In another aspect, the invention relates to a cell extract or a culture supernatant comprising the levansucrase and/or the endolevanase according to the invention.

In a further aspect, the invention is directed to a prebiotic or a food supplement comprising or (essentially) consisting of the fructooligosaccharides obtainable by the method for preparing fructooligosaccharides according to the invention.

A prebiotic or food supplement of the present invention can be comprised, for example, without being limited to it, in general food products, baby food and animal food.

All items, embodiments and examples described for the method for preparing fructooligosaccharides according to the invention also apply to the method for preparing levan, the fructooligosaccharides, the expression vector, the genetically modified host organism, the cell extract, the culture supernatant and the prebiotic or food supplement according to the invention, and vice versa.

### EXAMPLES

### Identification of a suitable levan-forming organism

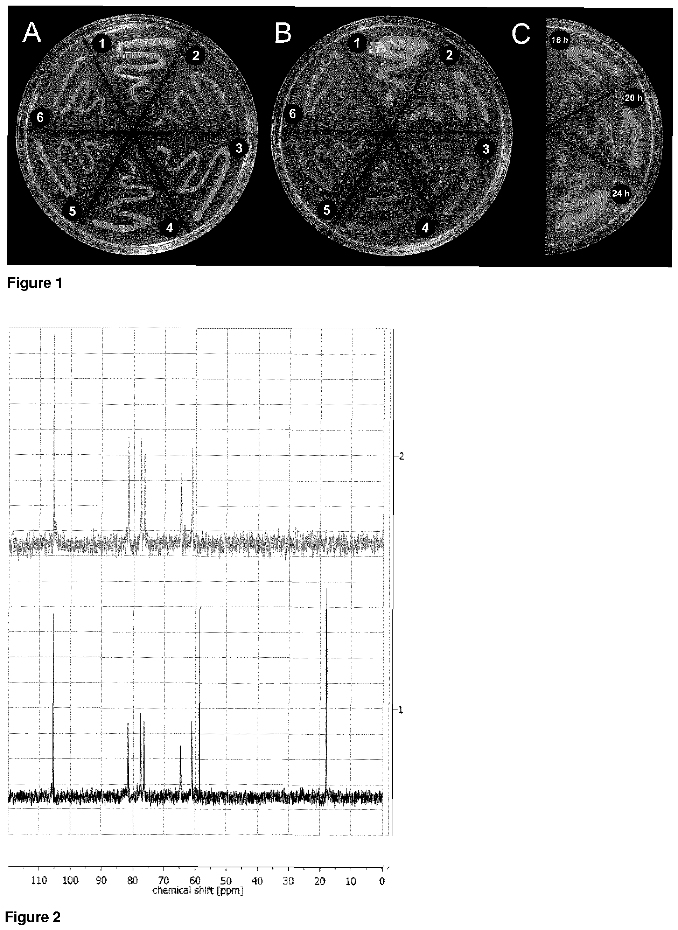

For the screening for potent levan producers, a total of six *Gluconobacter* strains were plated on yeast extract agar to which either mannitol or sucrose was added as a carbon source. The colony morphology after 24 hours of incubation at 28 °C was documented photographically and is shown in Figure 1. The production of extracellular polymeric substances (EPS) by microorganisms leads to a slimy colony morphology.

The screening shown in Figure 1 contributed to the identification of a potent producer of an extracellular polymeric substance. *G. japonicus* LMG 1417 (NCBI-Accession: SAMN03799280) showed a strong mucus production in the presence of sucrose, which is particularly evident in the time-lapse image (Figure 1C).

To identify the EPS produced by the organism, the polymer was isolated from the corresponding culture supernatant by ethanol precipitation. For this purpose, *G. japonicus* LMG 1417 was cultured in a complex medium consisting of yeast extract (6 g/L), sucrose (200 mM) and mannitol (5 mM). For buffering, a potassium phosphate buffer (pH 6.9) in a final concentration of 100 mM was added to the medium. After 24 hours of cultivation at 28 °C and 180 rpm shaking speed, the cells were separated by centrifugation and the culture supernatant mixed with three parts of 96 % ethanol. Ethanol precipitation is a cost-effective and reliable method for the precipitation of microbial polysaccharides (Smith et al. (2007) J. Chem. Technol. Biotechnol. 32:119-129., doi: 10.1002/jctb.5030320116). The precipitate was air-dried and then analyzed by ¹³C-NMR analysis and FTIR spectroscopy. As a reference for the analytical investigations commercial levan was used, which was produced by *Erwinia herbicola* and obtained from Sigma-Aldrich (Steinheim, Germany) (Blake et al. (1982) J. Bacteriol.). The spectra of the ¹³C-NMR analysis are shown in Figure 2.

The ¹³C-NMR analysis verified that the EPS produced by *G. japonicus* LMG 1417 is the prebiotic fructan-polymer levan. In addition to the six carbon signals (Table 1) that could be assigned to the monomeric fructose unit of levan, two additional signals with a shift of 58.72 ppm and 18.06 ppm were detected in the EPS spectrum (Figure 2). These signals were assigned to the precipitation reagent ethanol and indicated an incomplete drying of the levan preparation (Gottlieb et a. (1997) J. Org. Chem., doi: 10.1021/jo971176v).

**Table 1: ¹³C-NMR shifts of the examined levan preparations.**

| Carbon number | Chemical shift (ppm) Levan LMG 1417 | Chemical shift (ppm) Levan *E. herbicola* |
|---|---|---|
| C-1 | 61.11 | 61.12 |
| C-2 | 105.52 | 105.51 |
| C-3 | 77.51 | 77.51 |
| C-4 | 76.46 | 76.45 |
| C-5 | 81.61 | 81.60 |
| C-6 | 64.70 | 64.70 |

To substantiate the results of the ¹³C-NMR analysis, the two levan preparations were additionally analyzed by FTIR spectroscopy. In this method, the functional groups of the analyzed molecules are stimulated by long-wave infrared radiation, resulting in substance-specific absorption spectra. The two recorded spectra are shown in Figure 3.

The FTIR spectra of the levan preparations confirmed the assumption that the EPS formed by *G.japonicus* LMG 1417 is levan. In addition to the direct comparison of the preparations, the two spectra were also evaluated according to the publication of Barone and Medynets, in which an FTIR analysis of levan was carried out for the first time (Barone et al. (2007) Carbohydr. Polym., doi: 10.1016/j.carbpol.2007.01.017).

### Characterization of the levansucrase LevS1417 from G. japonicus LMG 1417

For detailed characterization of the levansucrase (LevS1417) from *G. japonicus* LMG 1417, the gene sequence coding for the enzyme (GenBank accession: KXV23964.1) was introduced into the overexpression vector pASK-IBA5plus (IBA GmbH). The native nucleotide sequence of the gene encoding for the levansucrase (LevS1417) from *G. japonicus* LMG 1417 is set forth in SEQ ID NO:4. The corresponding amino acid sequence of the native levansucrase from *G*. *japonicas* LMG 1417 is set forth in SEQ ID NO:2.

**Table 2: Oligonucleotide primers used to amplify the insert DNA.**

| Primer | Sequence (Restriction site marked in bold) | endonuclease |
|---|---|---|
| p5_*levS1417_*for | ATTA**CCGCGG**AAAATGCTATTTCCAGCCGAA | *Sac*II |
| p5_*levS1417_rev* | ATTA**CTCGAG**TCAGGCACGAACGTCATAGG | *Xho*I |

The primer sequences of p5_*levS1417*_for and p5*_levS1417*_rev are set forth in SEQ ID Nos. 9 and 10. The insertion was carried out using the endonucleases *Sac*II and *Xho*I*.* Due to the chosen cloning strategy, the N-terminus of the levansucrase was fused to a Strep-Tag II. This affinity tag enables efficient chromatographic purification of the recombinant protein from the total cell extract of an *E. coli* overexpression culture. The plasmid map of pASK-IBA5plus (IBA GmbH) is illustrated together with the components of the vector, for example, under: https://search.cosmobio.co.jp/cosmo_search_p/search_gate2/docs/IBA_/21404000.20060609.pdf.

The vector pASK5-IBA5plus (IBA GmbH) is designed for heterologous overproduction of proteins in *E. coli.* The Tet promoter upstream of the multiple cloning site (MCS) allows strong transcription of any gene insert. The promoter is regulated and can be activated by the addition of the inductor anhydrotetracycline. The final plasmid pASK5_levS1417 is shown in Figure 4.

The native sequence of the gene, coding for the levansucrase was altered by the cloning strategy and the associated modification of the 5'-end. The open-reading frame (ORF) coding for the modified levansucrase (Strep-Tag II, linker) is set forth in SEQ ID NO:3. The amino acid sequence of the modified levansucrase variant (N-terminal modification: Strep-Tag II, linker) is set forth in SEQ ID NO:1.

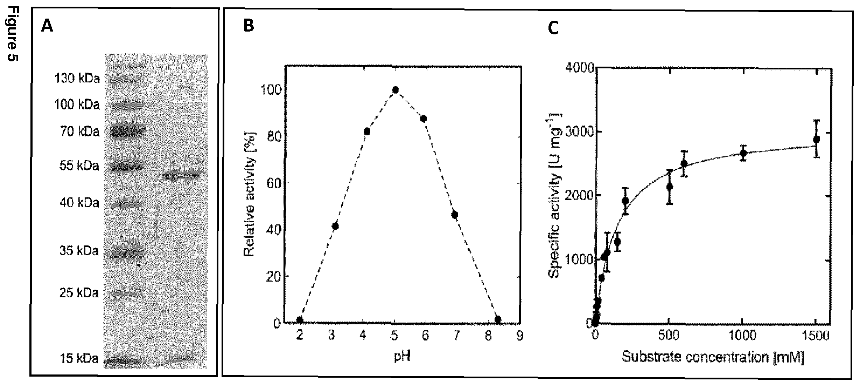

Following the heterologous overproduction of LevS1417 in *E. coli* DH5α, the levansucrase was purified from the total cell extract via the fused N-terminal Strep Tag II. The Strep-Tactin®XT Superflow ® 50 % suspension (IBA GmbH) served as the matrix for the chromatographic purification. The generated elution fraction was then separated by SDS-PAGE and proteins in the fraction were visualized by silver staining (Blum et al. 1987). For characterization of the levansucrase, a pH profile and Michaelis Menten kinetics were prepared for the recombinant enzyme. The images resulting from this characterization are shown in Figure 5.

The silver staining of the SDS gel confirmed the successful and reliable plasmid-mediated production of the recombinant levansucrase LevS1417 in *E. coli* DH5α. The protein, which has a predicted size of 51.2 kDa, was clearly visualized without any impurities. The pH profile shows that the investigated levansucrase is adapted to a slightly acidic environment and works optimally at a pH of 5. This observation coincides with the physiology of members of the genus *Gluconobacter,* which are adapted to an acidic habitat (Matsushita et al. (1989) Agric. Biol. Chem., doi: 10.1080/00021369.1989.10869793).

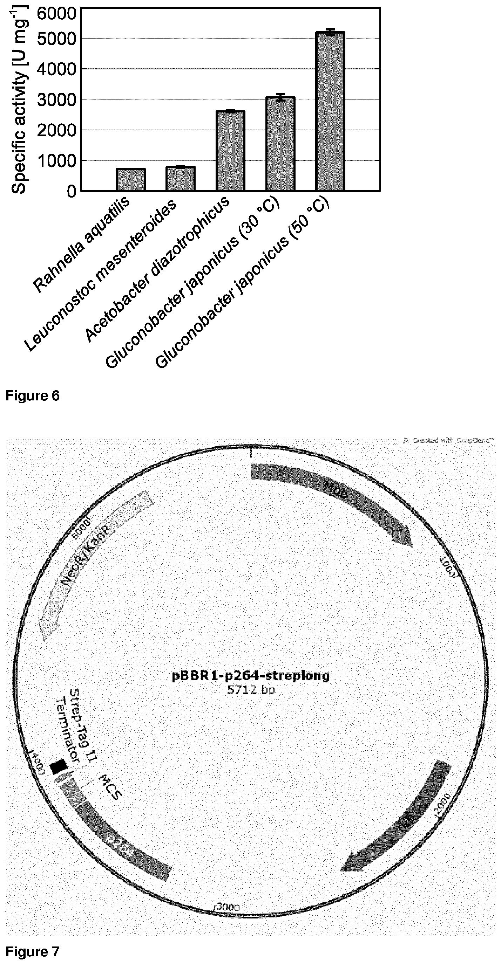

The enzyme behaves kinetically according to Michaelis-Menten and shows a Vₘₐₓ of 3064 ± 103 U mg⁻¹ and a Kₘ value of 147 ± 16 mM at 30 °C. At 50 °C a specific activity of 5190 ± 886,9 U mg⁻¹ was measured. As the comparative visualization of various levansucrase activities in Figure 6 shows, LevS1417 from *G. japonicus* LMG 1417 is the most active levansucrase described to date in the literature.

The enzyme is suitable for industrial applications due to its enormously high activity and was therefore selected as the basis for the intended production of levan-based FOS.

### Development of a cell-free levan production process based on G. japonicus LMG 1417

The *in-vitro*-characterization of the highly active levansucrase from *G*. *japonicus* LMG 1417 enabled two potential strategies for the production of levan-based FOS.
1) Initial *in*-*vivo*-production of high-molecular levan chains using *G*. *japonicus* LMG 1417 and subsequent hydrolysis of the polymer using a suitable endolevanase (e.g. in purified form, immobilized or as a cell extract).
2) Initial *in*-*vitro*-production of high molecular levan chains by the use of an *E. coli* cell extract containing the levansucrase from *G*. *japonicus* LMG 1417 and subsequent hydrolysis of the polymer by the use of a suitable endolevanase (e.g. in purified form, immobilized or as a cell extract).

In order to optimize the first strategy, a mutant strain based on *G. japonicus* LMG 1417 was generated, which is capable of a plasmid-mediated homologous overproduction of the investigated levansucrase LevS1417. The vector pBBR1-p264-streplong served as the platform for the overexpression (Zeiser et al. (2014) Appl. Microbiol. Biotechnol., doi: 10.1007/s00253-013-5016-5). This modified variant of the broad host range vector pBBR1MCS-2 was extended upstream of the MCS by a *Gluconobacter*-specific promoter region (p264). An additional insert downstream of the MCS contains the sequence coding for Strep-Tag II and the termination sequence of the pASK-IBA3 vector (IBA GmbH). The plasmid map of the generated vector is shown in Figure 7.

The mentioned promoter region is the upstream region of the gene gox0264 encoded in the genome of *Gluconobacter oxydans* 621H. A strong, constitutive promoter is located in this area. For homologous overexpression, the gene sequence coding for the levansucrase from *G. japonicus* LMG 1417 (GenBank accession: KXV23964.1) was amplified using the primers listed in Table 3.

**Table 3: Oligonucleotide primers used to amplify the insert DNA.**

| Primer | Sequence (Restriction site marked in bold) | Endonuclease |
|---|---|---|
| *levS1417_Eco*RV_for | ATTAGATATCAGGAGGAAAAAAAAATGAATGCTATTT CCAGC | *Eco*RV |
| *levS1417_Asc*I_rev | ATTA**GGCGCGCC**TCAGGCACGAACGTCATA | *Asc*I |

The primer sequences of *levS1417_Eco*RV_for and *levS1417_Asc*I_rev are set forth in SEQ ID Nos. 11 and 12.

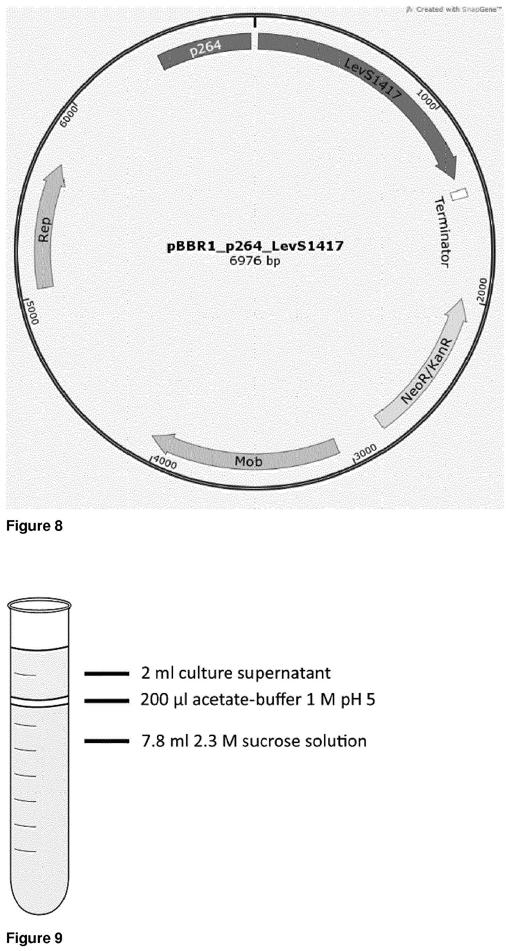

The amplificate was inserted via the restriction endonucleases *Eco*RV and *Asc*I into the complementarily digested vector pBBR1-p264-streplong. The insert was cloned off-frame to the Strep-Tag II coding sequence downstream of the MCS. The resulting plasmid pBBR1_p264_*levS1417* is shown in Figure 8.

The constructed plasmid was transformed by electroporation into electrocompetent cells of the strain *G. japonicus* LMG 1417. The transformation was carried out according to the protocol of Mostafa and colleagues (Mostafa et al. (2002) Appl. Environ. Microbiol., doi: 10.1128/AEM.68.5.2619-2623.2002).

The wild type *G. japonicus* LMG 1417 and the mutant *G. japonicus* LMG 1417 pBBR1_p264_*levS1417* were successfully used for cell-free levan production. Therefore, the two strains were first cultivated in the following medium up to an OD₆₀₀ₙₘ of 2.
YPSM-50/5:
- 3 g L⁻¹ Casein peptone
- 5 g L⁻¹ Yeast extract
- 50 mM Sucrose
- 5 mM Mannitol
   → For buffering, MES buffer pH 6.5 in a final concentration of 100 mM was supplemented.

The cells were harvested after 24 hours of incubation at 28 °C and 180 rpm shaking speed. The cultures were centrifuged for 25 minutes at room temperature and 20,000 rpm. The resulting supernatant served as the starting point for cell-free levan production, which is shown schematically in Figure 9. During cultivation, *G. japonicus* LMG 1417 secretes the levansucrase LevS1417 via an unknown secretory system into the culture supernatant. The supernatant can therefore be used directly for levan production without additional purification processes.

Using high-performance liquid chromatography (HPLC), the process educts and products were quantitatively analyzed at regular intervals.

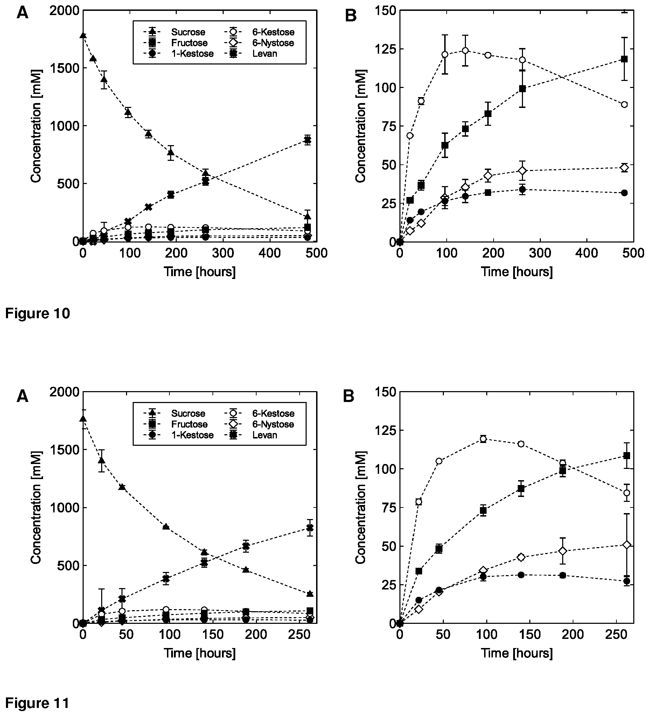

The reaction kinetics illustrated in Figure 10 shows that the supplemented sucrose was almost completely converted by the culture supernatant within 500 hours with levan as the major fructose-associated product.

In addition to levan and fructose, several FOS with varying degrees of polymerization were detected by HPLC. The loss of fructose units in the form of free fructose or FOS with a degree of polymerization of < 3 was 7.6 %. As expected, the majority of the fructose units contained in sucrose were incorporated into the high-molecular levan polymer. With a final levan concentration of 877 ± 42 mM (expressed in fructose equivalents), the levan yield of the described method is 157.9 ± 7.6 g L⁻¹. 90 % of the available sucrose was converted within 480 h.

To optimize the space-time yield of the process, the described overexpression mutant *G. japonicus* LMG 1417 pBBR1_p264_*levS1417* was constructed. The plasmid-mediated overproduction was intended to elevate the amounts of secreted levansucrase and thus enable faster conversion of the supplemented sucrose. The reaction course of the cell-free levan production based on the culture supernatant of the mutant strain is shown in Figure 11.

Using the culture supernatant of the overexpression mutant, a total of 7.2 % of the available fructose units were lost in the form of free fructose or FOS with a degree of polymerization of < 3. Again, as expected, the majority of the fructose units contained in the supplemented sucrose were incorporated into the high-molecular levan polymer. With a final levan concentration of 824 ± 72 mM (expressed in fructose equivalents), the levan yield of the described method was 148.3 ± 12.9 g L⁻¹. The time required for sucrose conversion was halved from about 490 to 260 hours compared to the wild type based process. The plasmid-mediated homologous overproduction of the levansucrase almost doubled the space-time yield of the cell-free process.

Since only one-fifth of the reaction mixture shown in Figure 9 consists of culture supernatant, one liter of *G. japonicus* LMG 1417 culture supernatant can be used for five liters of the described reaction. Thus, the maximum levan yield that can be obtained using one liter of the corresponding culture supernatant is 789.5 ± 38 g using the wild type culture supernatant. By using the culture supernatant of the overexpression mutant, 741.6 ± 64.8 g levan can be produced correspondingly.

Both systems thus deliver a comparable levan yield. Also, significant optimization of the space-time yield could be achieved by the plasmid-mediated homologous overproduction of the levansucrase. As Figure 10 shows, the levansucrase from *G. japonicus* LMG 1417 is an extremely stable enzyme that remains active even after almost 500 hours of incubation. However, the process based on the culture supernatant of the overexpression mutant was completed after 260 hours. This means that the proportion of the mutant supernatant in the process solution could be halved and complete conversion of the added sucrose would still be achieved. Thus, starting from one liter of culture of the mutant *G*. *japonicus* LMG 1417 pBBR1_p264_*levS1417*, 10 liters of the process solution could be prepared. The theoretical levan yield of this process would be about 1.5 kg.

Because the cell-free process can bypass the complicated and time-consuming separation of bacterial cells from the highly viscous levan solution, it becomes clear that the developed system represents a fundamental improvement over the cell-based methods described in the literature.

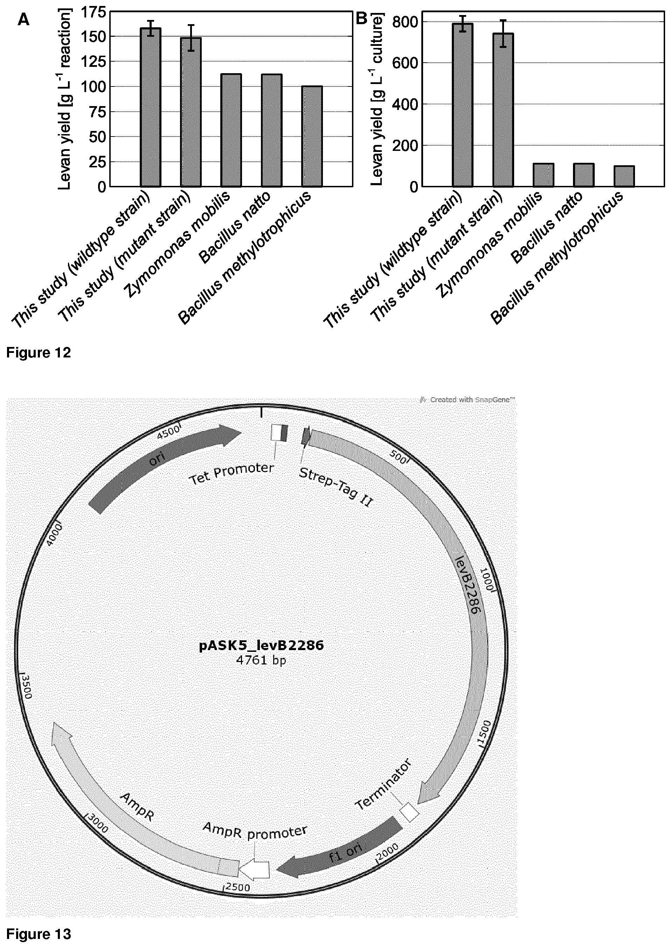

For a comparison of the developed cell-free method with literature values, Figure 12 shows the most productive processes based on *Zymomonas mobilis* CCT 4494 (Lorenzetti et al. (2015) J. Food Process Eng. 38:31-36., doi: 10.1111/jfpe.12123), *Bacillus subtilis* (*natto*) CCT7712 (Dos Santos et al. (2013) Rom. Biotechnol. Lett.) and *Bacillus methylotrophicus* SK 21.002 (Zhang et al. (2014) Carbohydr. Polym., doi: 10.1016/j.carbpol.2013.10.045).

### Identification and characterization of suitable endolevanases

With G. *japonicus* LMG 1417 an extremely potent basis for the intended production of levan-based FOS could be identified and characterized. The findings enabled two production strategies for the production of the prebiotic polymer levan.
1) Enzymatic production using the high-active levansucrase from *G. japonicus* LMG 1417 (e.g. in purified form or as *E. coli cell* extract)
2) A production based on the culture supernatant of *G. japonicus* LMG 1417 (e.g. wild type or overexpression mutant)
An enzymatic strategy based on the use of a suitable endolevanase was selected for the intended production of short-chain levan-type FOS. Enzyme class EC 3.2.1.65 endolevanases are capable of hydrolyzing levan into short-chain FOS. For a detailed characterization, a total of three endolevanases were cloned into independent overexpression vectors.
- BT1760 from *Bacteroides thetaiotaomicron* DSM 2079
- LevB1 from *Bacillus licheniformis* DSM 13
- LevB2286 from *Azotobacter chroococcum* DSM 2286

Again, the vector pASK-IBA5plus (IBA GmbH) served as the basis for the heterologous production of the desired endolevanases in *E. coli.* In the following, particular focus will be placed on the previously uncharacterized endolevanase from *A. chroococcum* DSM 2286. The gene sequence coding for the endolevanase was amplified using the oligonucleotide primers listed in Table 4. The selected cloning strategy deleted the native N-terminal signal peptide of the endolevanase.

**Table 4: Oligonucleotide primers used to amplify the insert DNA.**

| Primer | Sequence | Endonuclease |
|---|---|---|
| *levB2286*_p5_f | ATGGTAGGTCTCAGCGCCCCGCTCCGGCGACCCC G | *Bsa*I |
| *levB2286*_p5_r | ATGGTAGGTCTCATATCAGTCTGCGGGCGCCGCTG GC | *Bsa*I |

The primer sequences of *levB2286*_p5_f and *levB2286*_p5_r are set forth in SEQ ID Nos. 13 and 14.

The amplificate was introduced via the restriction interfaces Bsal into the complementarily digested vector pASK-IBA5plus.

The resulting overexpression plasmid pASK5_*levB2286* is shown in Figure 13.

The native sequence of the endolevanase (SEQ ID NO:8) was altered by the cloning strategy. The open-reading frame (ORF) coding for the modified endolevanase has the sequence set forth in SEQ ID NO:7 (modification: sequence coding for Strep-Tag II; linker). The corresponding amino acid sequence of the modified endolevanase variant (N-terminal modification: Strep-Tag II; linker) is set forth in SEQ ID NO:5. The native amino acid sequence is set forth in SEQ ID NO:6.

Following plasmid-mediated heterologous overexpression of the endolevanase from *A. chroococcum* DSM 2286 in *E. coli* DH5α, the recombinant protein was purified from the cell extract by streptactin affinity chromatography. The successful production and purification of the recombinant protein was verified after the separation of the elution fraction via SDS-PAGE. The 59.2 kDa protein was clearly visualized by silver staining. Characterization of the purified enzyme was carried out, with particular attention to the activity and the specific product spectrum. For the intended process, the investigated endolevanase should ideally generate FOS with a degree of polymerization (DP) of ≥ 3. According to Regulation (EU) No 1169/2011 of the European Parliament, carbohydrate polymers consisting of three or more monomeric units can be declared as fiber. Dietary fibers are significantly more valuable than di- or monosaccharides and are therefore desirable reaction products.

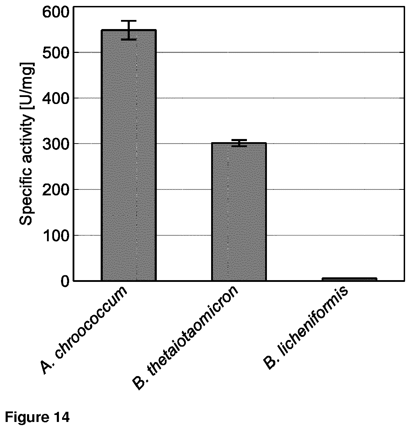

As Figure 14 shows, the endolevanase from *A. chroococcum* DSM 2286 is the fastest endolevanase, which has been described within the enzyme class EC 3.2.1.65 to date. At 30 °C, the enzyme has a specific activity of ∼ 550 U mg⁻¹.

An important criterion for the selection of an endolevanase for the intended production of levan-based FOS was, in addition to high activity, the specific product spectrum of the corresponding enzyme. For this purpose, the FOS formed during the enzymatic reaction were quantified by HPLC to determine the proportion of desired FOS with a degree of polymerization of ≥ 3.

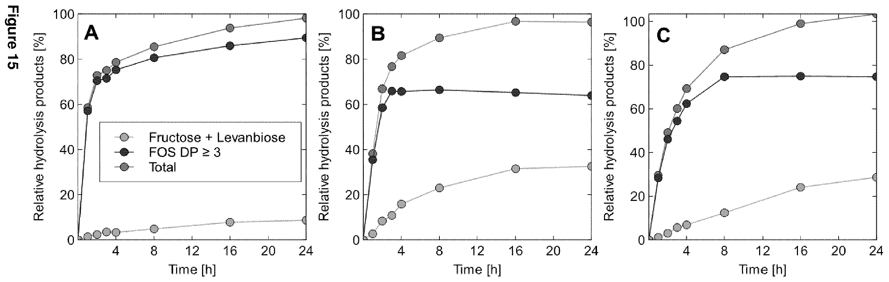

The product analysis of the various endolevanases illustrated in Figure 15 shows that the endolevanase from *A. chroococcum* DSM 2286 almost exclusively produces FOS with a degree of polymerization of ≥ 3. Thus, the reaction products according to Regulation (EU) No 1169/2011 of the European Parliament are high-quality and prebiotic dietary fibers. To date, no enzyme has been described that generates such a high proportion of dietary fiber from levan. Also, the enzyme has a very low affinity for the dietary fibers produced in the course of the enzymatic reaction, since the concentration of the desired FOS does not decrease even in the late course of incubation.

This observation is unique to date, as previously characterized endolevanases further degrade long-chain FOS, generating large amounts of fructose and levanbiose. This hydrolysis behavior was also observed using the endolevanases from *Bacteroides thetaiotaomicron* DSM 2079 and *Bacillus licheniformis* DSM 13, as Figure 15 shows. Both enzymes produce large amounts of fructose and levanbiose with increasing incubation time. Mardo and colleagues were able to show that the endolevanase from *Bacteroides thetaiotaomicron* DSM 2079 releases up to 50 % of the fructose units contained in the levan in the form of free fructose after prolonged incubation (Mardo et al. (2017) PLoS One 12, doi: 10.1371/journal.pone.0169989). The endolevanase from *Bacillus licheniformis* IBt1 also forms levanbiose as a primary hydrolysis product during prolonged incubation, which cannot be declared as prebiotic dietary fiber (Porras-Domínguez et al. (2014) Process Biochem., doi: 10.1016/j.procbio.2014.02.005).

The investigated endolevanase from *A. chroococcum* DSM 2286 is the only endolevanase described so far that guarantees a unique combination of high activity and high product stability. The enzyme is therefore ideally suited for the industrial production of levan-based dietary fibers.

### Construction of an extract-based production process for levan-type FOS

The studies on the levansucrase from *G. japonicus* LMG 1417 and endolevanase from *A. chroococcum* DSM 2286 showed that both enzymes can be produced in high amounts in *E. coli.* A coupling of the catalytic properties of both proteins enables the production of levan-based FOS in a single reaction approach starting from sucrose.
In order to enable simultaneous production of both proteins in a single *E. coli* strain, an overexpression plasmid was generated that carries the coding genes of both proteins.
The plasmid pASK5_*levS1417*, which was constructed for the overexpression of the levansucrase from *G.* japonicus LMG 1417, served as the starting point for the cloning strategy. Using the oligonucleotide primers listed in Table 5, the gene sequence coding for the levansucrase was amplified together with the flanking regulatory elements (promoter region and termination sequence).

**Table 5: Oligonucleotide primers used to amplify the insert DNA.**

| Primer | Sequence |
|---|---|
| *levS1417*_Assembly_f | GACCCGACACCATCGAATGGATTAATTCCTAATTTTTGTTGACACTC |
| *levS1417*_Assembly_r | ATTAGGAATTAATCATCTGGAGATCCGTGACGCAGTAG |

The primer sequences of *levS1417*_Assembly_f and *levS1417*_Assembly_r are set forth in SEQ ID Nos. 15 and 16.

The amplificate was inserted by using the NEBuilder® HiFi DNA Assembly Master Mix into the plasmid pASK5_*levB2286*, which was previously enzymatically linearized via the endonuclease *Msc*I.

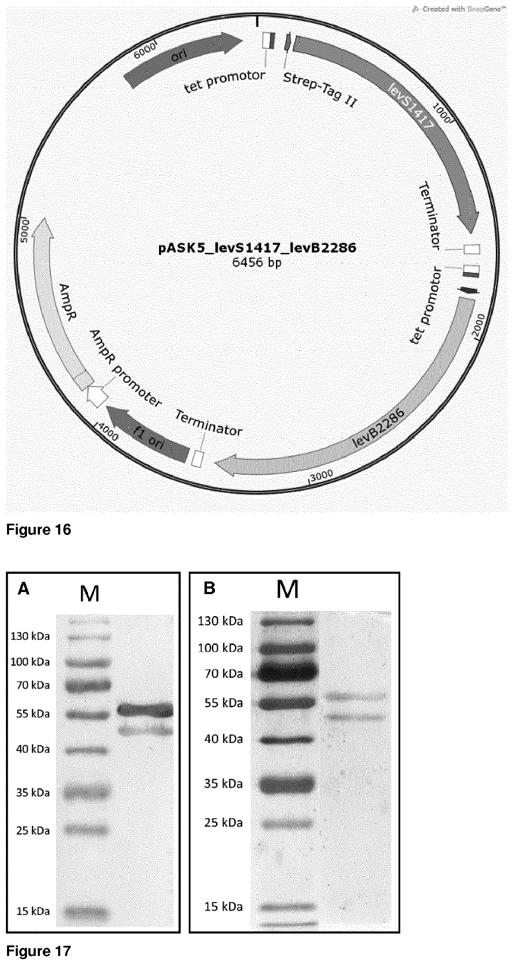

The resulting plasmid pASK5_levS1417_levB2286 is shown in Figure 16.

After the overexpression plasmid was introduced into *E. coli* BL21, the recombinant proteins were purified by affinity chromatography after heterologous production. The proteins in the elution fraction were visualized by SDS-PAGE and silver staining.

Both the western blot and silver staining carried out following overproduction revealed the successful simultaneous production of the two recombinant proteins. The bands shown in Figure 17 could be assigned to the levansucrase (51.2 kDa) and the endolevanase (59.3 kDa).

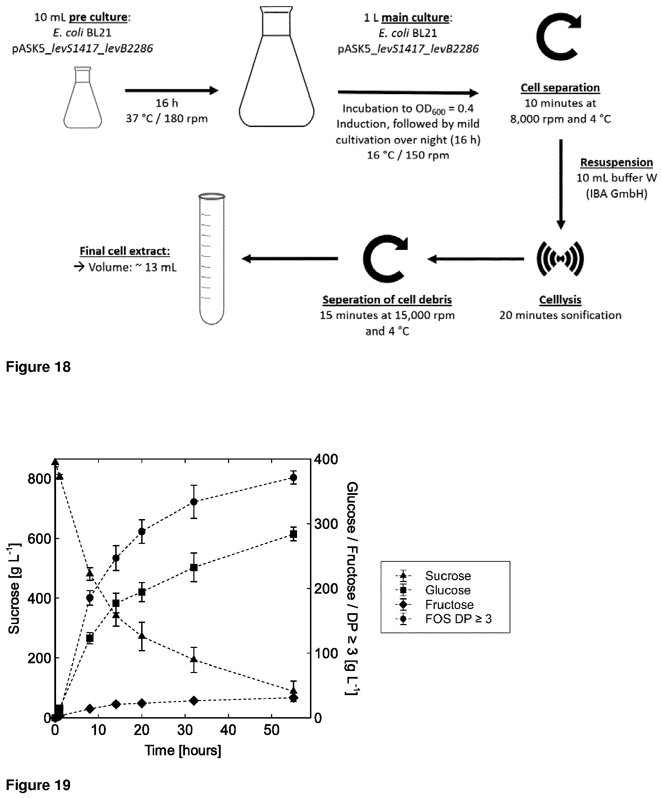

After the production strain was verified by biochemical methods, the functionality of the recombinant proteins had to be investigated. An assay based on cell extract of the generated overexpression strain was developed for this purpose. The use of cell extract avoids the costly and time-consuming purification of the recombinant proteins. Figure 18 shows in simplified form the production of the raw extract.

In order to validate whether sucrose can be enzymatically converted into levan-based FOS in a single reaction, a suitable assay based on the *E. coli* cell extract was performed. A saturated sucrose solution (∼ 2.5 M) was adjusted to pH 5 by adding a sodium acetate buffer (40 mM), which ensures the functionality of both enzymes. The enzymatic reaction was started by adding the cell extract. The added extract volume corresponded to 3.85 mL per L reaction. The educts and products were again quantified by HPLC (Figure 19).

Supplemented sucrose was almost completely converted within 55 hours. Only 7.8 ± 0.2 % of the fructose units contained in the sucrose were released in the form of free fructose. Most of the fructose was introduced into the levan polymer by the recombinant levansucrase, which was then hydrolyzed to short-chain FOS by the catalytic activity of the recombinant endolevanase. After 55 hours of incubation, a FOS yield of 371.7 ± 10.2 g L-1 was detected. The described process converted the fructose units contained in sucrose to FOS with a DP of ≥ 3 with an efficiency of 88.4 %. The fructose concentration at this time (t₅₅ₕ) was 173 ± 6 mM. A concentration of 43 ± 9 mM could be determined for levanbiose. The loss of fructose units that were not incorporated into FOS with a DP of ≥ 3 was thus 11.6 %.

One liter of the *E. coli* cell extract described in Figure 18 can thus generate ∼ 320 kg of levan-based dietary fiber from sucrose in a single reaction. A comparable yield could not be achieved with any of the methods described in the literature.

### Activity Assays

For activity assays, the two described enzymes (levansucrase and endolevanase) were heterologously overproduced in *Escherichia coli* DH5α and subsequently purified by affinity chromatography. The purification was carried out via the N-terminal Strep-Tag II using StrepTactin®XT Superflow® (IBA GmbH).

### Levansucrase-activitv-assav:

Detailed Michaelis-Menten kinetics were established for the levansucrase from *Gluconobacter japonicus* LMG 1417. Using non-linear regression, the following catalytic properties were determined for the enzyme:
- Vₘₐₓ = 3064 ± 103 U mg⁻¹
- K_{M} = 147 ± 16 mM

The assays to determine the specific levansucrase activity were performed at 30 °C. The reaction was buffered by a sodium acetate buffer pH 5.4 at a final concentration of 100 mM. Sodium-acetate buffer (10x Stock / 100 mL):
- 86 mL 1 M Sodium-acetate
- 14 mL 1M Acetic acid

Sucrose in different concentrations (0-1500 mM) served as the substrate for the reactions.

### Endolevanase-activity assay:

The specific activity of the endolevanase (1001.9 U mg⁻¹) from *Azotobacter chroococcum* DSM 2286 was measured at 30 °C. The reaction was buffered by a Mcllvaine buffer pH 6.

Mcllvaine (Phosphate-Citrate) Puffer (10x Stock / 100 mL):
- 36,85 mL 1 M Citric acid
- 63,15 mL 2 M Na₂HPO₄

For the described reaction, levan in a final concentration of 260 mM (expressed in fructose equivalents) served as substrate.

SEQUENCE LISTING

<120>  ENZYMATIC PRODUCTION OF LEVAN-BASED, PREBIOTIC
       FRUCTOOLIGOSACCHARIDES

<130>  P73645

<160>  16

<170>  PatentIn version 3.5

<210>  1
<211>  461
<212>  PRT
<213>  Artificial

<220>
<223>  Amino acid sequence of the modified levansucrase from
       Gluconobacter japonicus LMG 1417

<400>  1

Met Ala Ser Trp Ser His Pro Gln Phe Glu Lys Gly Ala Glu Thr Ala
1               5                   10                  15

Glu Asn Ala Ile Ser Ser Arg Ile Leu Asp Gln His Met Ala Val Arg
            20                  25                  30

Arg Val Leu Pro Asn Tyr Arg Ser Ser Arg Trp Thr Ile Ala Asp Ala
        35                  40                  45

Leu Lys Val Arg Thr Asp Asp Pro Thr Thr Thr Met Pro Val Ile Asp
    50                  55                  60

Tyr Ser Phe Pro Val Met Ser Asn Asp Val Met Ile Trp Asp Ser Gly
65                  70                  75                  80

Ala Leu Arg Asp Met His Met Arg Thr Val Thr Tyr Lys Gly Trp Phe
                85                  90                  95

Val Leu Trp Ser Leu Ala Val Asp Lys Ile Asp Lys Thr Asn Ala Lys
            100                 105                 110

Ile Tyr Asp Glu Trp His Ser Arg Asn Asp Arg Ala Tyr Ile Gly Tyr
        115                 120                 125

Trp Tyr Ser Ala Asp Gly Val Glu Trp Thr Trp Gly Gly Arg Leu Phe
    130                 135                 140

Gln Thr Ser Ala Asp Leu Arg Pro Trp Glu Trp Ser Gly Ser Leu Val
145                 150                 155                 160

Met Arg Glu Gly Thr Glu Asn Lys Val Asp Met Phe Tyr Thr Ser Val
                165                 170                 175

Gly Ala Pro Asp Gly Asn Ser Val Pro Ala Val Cys Ser Gly Thr Ile
            180                 185                 190

His Ala Asp Asp Lys Gly Val Trp Phe Glu Gly Phe Ser Ala Ser Thr
        195                 200                 205

Glu Met Phe Lys Ala Asp Gly Val His Tyr Ala Asn Ala Ser Glu Asp
    210                 215                 220

Ala Tyr Phe Asp Phe Arg Asp Pro Gln Pro Phe Ile Asn Pro Gly Asp
225                 230                 235                 240

Gly Glu Leu Tyr Thr Leu Phe Glu Gly Asn Val Pro Gly Met Arg Gly
                245                 250                 255

Gln Phe Val Val Gly Ser Asp Glu Met Gly His Val Pro Pro Gly Tyr
            260                 265                 270

Ala Val Asp Pro Gly Ala Gln Tyr Gly Ala Ala Ala Ile Gly Leu Ser
        275                 280                 285

Arg Cys Val Ser Asn Trp Arg Lys Gly Asp Tyr Ser Arg Trp Glu Leu
    290                 295                 300

Met Pro Ala Leu Val Thr Ala Leu Gly Val Asn Asp Gln Thr Glu Arg
305                 310                 315                 320

Pro His Phe Val Phe Lys Asp Gly Leu Thr Tyr Leu Phe Thr Ile Ser
                325                 330                 335

His His Ser Thr Tyr Thr Gly Lys Ser Thr Gly Pro Asp Gly Val Tyr
            340                 345                 350

Gly Phe Val Ser Glu Lys Gly Ile Phe Gly Pro Tyr Lys Pro Leu Asn
        355                 360                 365

Ser Ser Gly Leu Val Leu Gly Asn Pro Ser Ser Gln Pro Tyr Ser Thr
    370                 375                 380

Tyr Ser His Phe Val Asp Ala Gln Gly Tyr Val Gln Ser Phe Ile Asp
385                 390                 395                 400

Thr Ile Pro Ala Ala Gly Gln Asn Ala Glu Ser Pro Asp Thr Tyr Arg
                405                 410                 415

Ile Gly Gly Thr Leu Ala Pro Thr Val Arg Leu Val Leu Asp Gly Asp
            420                 425                 430

Arg Thr Phe Leu Thr Glu Val His Asp Tyr Ala Gln Val Phe Ala Gln
        435                 440                 445

Ala Ala Trp Pro Ala Ser Leu Ala Tyr Asp Val Arg Ala
    450                 455                 460

<210>  2
<211>  445
<212>  PRT
<213>  Amino acid sequence of the levansucrase from Gluconobacter japonicus
LMG 1417

<400>  2

Met Asn Ala Ile Ser Ser Arg Ile Leu Asp Gln His Met Ala Val Arg
1               5                   10                  15

Arg Val Leu Pro Asn Tyr Arg Ser Ser Arg Trp Thr Ile Ala Asp Ala
            20                  25                  30

Leu Lys Val Arg Thr Asp Asp Pro Thr Thr Thr Met Pro Val Ile Asp
        35                  40                  45

Tyr Ser Phe Pro Val Met Ser Asn Asp Val Met Ile Trp Asp Ser Gly
    50                  55                  60

Ala Leu Arg Asp Met His Met Arg Thr Val Thr Tyr Lys Gly Trp Phe
65                  70                  75                  80

Val Leu Trp Ser Leu Ala Val Asp Lys Ile Asp Lys Thr Asn Ala Lys
                85                  90                  95

Ile Tyr Asp Glu Trp His Ser Arg Asn Asp Arg Ala Tyr Ile Gly Tyr
            100                 105                 110

Trp Tyr Ser Ala Asp Gly Val Glu Trp Thr Trp Gly Gly Arg Leu Phe
        115                 120                 125

Gln Thr Ser Ala Asp Leu Arg Pro Trp Glu Trp Ser Gly Ser Leu Val
    130                 135                 140

Met Arg Glu Gly Thr Glu Asn Lys Val Asp Met Phe Tyr Thr Ser Val
145                 150                 155                 160

Gly Ala Pro Asp Gly Asn Ser Val Pro Ala Val Cys Ser Gly Thr Ile
                165                 170                 175

His Ala Asp Asp Lys Gly Val Trp Phe Glu Gly Phe Ser Ala Ser Thr
            180                 185                 190

Glu Met Phe Lys Ala Asp Gly Val His Tyr Ala Asn Ala Ser Glu Asp
        195                 200                 205

Ala Tyr Phe Asp Phe Arg Asp Pro Gln Pro Phe Ile Asn Pro Gly Asp
    210                 215                 220

Gly Glu Leu Tyr Thr Leu Phe Glu Gly Asn Val Pro Gly Met Arg Gly
225                 230                 235                 240

Gln Phe Val Val Gly Ser Asp Glu Met Gly His Val Pro Pro Gly Tyr
                245                 250                 255

Ala Val Asp Pro Gly Ala Gln Tyr Gly Ala Ala Ala Ile Gly Leu Ser
            260                 265                 270

Arg Cys Val Ser Asn Trp Arg Lys Gly Asp Tyr Ser Arg Trp Glu Leu
        275                 280                 285

Met Pro Ala Leu Val Thr Ala Leu Gly Val Asn Asp Gln Thr Glu Arg
    290                 295                 300

Pro His Phe Val Phe Lys Asp Gly Leu Thr Tyr Leu Phe Thr Ile Ser
305                 310                 315                 320

His His Ser Thr Tyr Thr Gly Lys Ser Thr Gly Pro Asp Gly Val Tyr
                325                 330                 335

Gly Phe Val Ser Glu Lys Gly Ile Phe Gly Pro Tyr Lys Pro Leu Asn
            340                 345                 350

Ser Ser Gly Leu Val Leu Gly Asn Pro Ser Ser Gln Pro Tyr Ser Thr
        355                 360                 365

Tyr Ser His Phe Val Asp Ala Gln Gly Tyr Val Gln Ser Phe Ile Asp
    370                 375                 380

Thr Ile Pro Ala Ala Gly Gln Asn Ala Glu Ser Pro Asp Thr Tyr Arg
385                 390                 395                 400

Ile Gly Gly Thr Leu Ala Pro Thr Val Arg Leu Val Leu Asp Gly Asp
                405                 410                 415

Arg Thr Phe Leu Thr Glu Val His Asp Tyr Ala Gln Val Phe Ala Gln

420                 425                 430

Ala Ala Trp Pro Ala Ser Leu Ala Tyr Asp Val Arg Ala
        435                 440                 445

<210>  3
<211>  1386
<212>  DNA
<213>  Artificial

<220>
<223>  Nucleotide sequence of the modified levansucrase gene from
       Gluconobacter japonicus LMG 1417

<400>  3
atggctagct ggagccaccc gcagttcgaa aaaggcgccg agaccgcgga aaatgctatt      60

tccagccgaa ttctggatca gcacatggct gttcgtcgcg tgcttccgaa ctatcgctcg     120

tcccggtgga cgattgctga tgcgctgaag gttcgtacgg acgatccgac gacgacaatg     180

ccggtaatcg actacagttt cccagtcatg tccaacgatg tgatgatctg ggacagtggc     240

gcccttcgtg acatgcatat gcgcactgtg acgtataagg gctggtttgt tctctggtct     300

ctggctgtgg acaaaatcga caagaccaat gccaagattt atgacgaatg gcactcccgc     360

aatgatcgcg cctatatcgg ctactggtac agcgccgacg gtgttgagtg gacatggggt     420

ggccgcctgt ttcagacgtc tgctgatctg cgtccctggg agtggtccgg ttctctggtg     480

atgcgtgaag gcaccgagaa caaggtcgac atgttctaca cgtccgttgg tgcgccggat     540

ggtaattctg ttccggctgt ttgctccggc accattcatg ctgatgacaa gggtgtctgg     600

tttgagggct tctcggccag cacggaaatg ttcaaggctg atggcgttca ttatgccaac     660

gcttctgaag atgcgtattt cgatttccgc gatccgcagc cgttcatcaa tccgggtgat     720

ggcgagcttt acacgctgtt cgaaggcaat gttcctggca tgcgggggca gtttgttgtc     780

ggttctgacg agatgggcca tgttccgcca ggttatgcgg tggatccggg tgcgcaatat     840

ggtgctgcgg caatcggtct ctcccggtgt gtgtctaact ggcgcaaggg tgattattcc     900

cgttgggagc tgatgcctgc tctggtgacg gctctgggcg tgaacgatca gacggagcgt     960

ccgcatttcg tcttcaagga cggtctgacc tatctgttca ccatcagcca tcattccacc    1020

tatacaggca aagtacgggc cctgatggtt gtgtatggtt tcgtgtccga gaaggggatt    1080

tttggtccct acaagcctct aaacagctcg ggtctggtac ttggtaaccc gtcttcacag    1140

ccgtattcga cgtattcgca ttttgtggat gcgcagggct acgtccagtc gtttatcgat    1200

acgattccgg cggctggtca gaacgctgaa agccctgata cctatcgtat tggcggcacg    1260

cttgctccga cggtgcggct ggttctggat ggtgatcgga cgttcctgac ggaagttcat    1320

gactatgcgc aggtttttgc gcaggccgcc tggccggctt ctctggccta tgacgttcgt    1380

gcctga                                                              1386

<210>  4
<211>  1338
<212>  DNA
<213>  Nucleotide sequence of the levansucrase gene from Gluconobacter
japonicus LMG 1417

<400>  4
atgaatgcta tttccagccg aattctggat cagcacatgg ctgttcgtcg cgtgcttccg      60

aactatcgct cgtcccggtg gacgattgct gatgcgctga aggttcgtac ggacgatccg     120

acgacgacaa tgccggtaat cgactacagt ttcccagtca tgtccaacga tgtgatgatc     180

tgggacagtg gcgcccttcg tgacatgcat atgcgcactg tgacgtataa gggctggttt     240

gttctctggt ctctggctgt ggacaaaatc gacaagacca atgccaagat ttatgacgaa     300

tggcactccc gcaatgatcg cgcctatatc ggctactggt acagcgccga cggtgttgag     360

tggacatggg gtggccgcct gtttcagacg tctgctgatc tgcgtccctg ggagtggtcc     420

ggttctctgg tgatgcgtga aggcaccgag aacaaggtcg acatgttcta cacgtccgtt     480

ggtgcgccgg atggtaattc tgttccggct gtttgctccg gcaccattca tgctgatgac     540

aagggtgtct ggtttgaggg cttctcggcc agcacggaaa tgttcaaggc tgatggcgtt     600

cattatgcca acgcttctga agatgcgtat ttcgatttcc gcgatccgca gccgttcatc     660

aatccgggtg atggcgagct ttacacgctg ttcgaaggca atgttcctgg catgcggggg     720

cagtttgttg tcggttctga cgagatgggc catgttccgc caggttatgc ggtggatccg     780

ggtgcgcaat atggtgctgc ggcaatcggt ctctcccggt gtgtgtctaa ctggcgcaag     840

ggtgattatt cccgttggga gctgatgcct gctctggtga cggctctggg cgtgaacgat     900

cagacggagc gtccgcattt cgtcttcaag gacggtctga cctatctgtt caccatcagc     960

catcattcca cctatacagg caaaagtacg ggccctgatg gtgtgtatgg tttcgtgtcc    1020

gagaagggga tttttggtcc ctacaagcct ctaaacagct cgggtctggt acttggtaac    1080

ccgtcttcac agccgtattc gacgtattcg cattttgtgg atgcgcaggg ctacgtccag    1140

tcgtttatcg atacgattcc ggcggctggt cagaacgctg aaagccctga tacctatcgt    1200

attggcggca cgcttgctcc gacggtgcgg ctggttctgg atggtgatcg gacgttcctg    1260

acggaagttc atgactatgc gcaggttttt gcgcaggccg cctggccggc ttctctggcc    1320

tatgacgttc gtgcctga                                                  1338

<210>  5
<211>  545
<212>  PRT
<213>  Artificial

<220>

<223>  Amino acid sequence of the modified endolevanase from Azotobacter
      chroococcum DSM 2286

<400>  5

Met Ala Ser Trp Ser His Pro Gln Phe Glu Lys Gly Ala Pro Ala Pro
1               5                   10                  15

Ala Thr Pro Ser Gly Pro Glu Pro Ser Ser Pro Pro Leu Thr Lys Pro
            20                  25                  30

Ser Ala Phe Ser Arg Pro Ala Asp Trp Ser Pro Tyr Arg Pro Ala Val
        35                  40                  45

His Leu Thr Pro Ala Lys His Trp Met Asn Asp Pro Gln Arg Pro Ile
    50                  55                  60

Leu Ile Asp Gly Val Trp His Tyr Tyr Tyr Leu Tyr Asn Ala Asp Tyr
65                  70                  75                  80

Pro Lys Gly Asn Gly Thr Glu Trp Tyr His Ala Thr Ser Thr Asp Leu
                85                  90                  95

Val His Trp Gln Asp His Gly Val Ala Ile Asp Lys Tyr Lys Asn Gly
            100                 105                 110

Leu Gly Asp Ile Glu Thr Gly Ser Ala Val Ile Asp Thr Lys Asn Thr
        115                 120                 125

Ala Gly Phe Gly Ala Gly Ala Val Ile Ala Val Met Thr Gln Gln His
    130                 135                 140

Glu Gly Val Gln Arg Gln Ser Leu Phe Val Ser Thr Asp Gly Gly Tyr
145                 150                 155                 160

Arg Phe Lys Ala Tyr Asp Gly Asn Pro Val Met Asp Asn Pro Gly Ala
                165                 170                 175

Glu His Trp Arg Asp Pro Lys Ile Ile Trp Asp Asp Ala Arg Asp Glu
            180                 185                 190

Trp Leu Met Ala Leu Ala Glu Gly Asp Lys Ile Gly Phe Tyr Thr Ser
        195                 200                 205

Pro Asp Leu Lys Arg Trp Thr Tyr Arg Ser Gly Phe Glu Arg Asp Asp
    210                 215                 220

Leu Gly Ile Leu Glu Cys Pro Asp Leu Phe Arg Met Ser Val Asp Gly
225                 230                 235                 240

Asp Pro Ala Asn Thr Arg Trp Val Leu Ala Ala Gly Gly Asn Gly Ala
                245                 250                 255

Ser Lys Gly Met Thr Thr Gly Thr Val Tyr Trp Thr Gly Asn Trp Asp
            260                 265                 270

Gly Glu Arg Phe Thr Ala Asp Arg Asp Glu Pro Arg Trp Leu Asp Ser
        275                 280                 285

Gly Ala Asp Phe Tyr Ala Thr Val Thr Trp Asp Asp Pro Arg Gln Asp
    290                 295                 300

Asp Ala Glu Arg Leu Ala Ser Arg Tyr Ala Ile Gly Trp Leu Asn Asn
305                 310                 315                 320

Trp Ala Tyr Ala Thr Lys Leu Pro Thr Gly Asp Trp His Gly Gly Ala
                325                 330                 335

Asp Ser Ile Val Arg Arg Ile Glu Leu Arg Ser Val Asp Gly Glu Pro
            340                 345                 350

Leu Leu Val Ser Gln Pro Val Asp Ala Leu Asp Lys Leu Ala Gly Asn
        355                 360                 365

Ala Glu Val Arg Ser Lys Val Arg Val Thr Ala Ala Ser Gly Thr Thr
    370                 375                 380

Leu Pro Gln Pro Glu Ser Asp Ala Tyr Arg Leu Gln Val Glu Leu Asp
385                 390                 395                 400

Ala Ala Ser Gly Ala Asn Glu Val Arg Phe Arg Val Lys Glu Gly Gly
                405                 410                 415

Gly His Phe Thr Thr Val Gly Tyr Asn Phe Val His Gly Val Ala Phe
            420                 425                 430

Val Ala Arg Ala Ala Asp Ala Ala Ala Glu Arg Met Pro Ala Val Tyr
        435                 440                 445

Arg Glu Val Arg Thr Ala Pro Ala Pro Ala Arg Asp Gly Thr Val Thr
    450                 455                 460

Leu Asp Val Ile Val Asp Ala Ala Ser Val Glu Val Phe Val Asn Asp
465                 470                 475                 480

Gly Glu Ala Val Leu Ser Asn Leu Ala Phe Gly Ala Pro Gly Ala Asn

485                 490                 495

Gly Leu Ser Val Glu Ser Leu Gly Gly Asp Thr Glu Leu Arg Ser Phe
            500                 505                 510

Arg Leu Thr Pro Leu Ala Ile Ala Pro Ile Glu Arg His Met Glu Gln
        515                 520                 525

Ala Pro Gln Gly Asn Gly Gly Ser Ala Gly Lys Pro Ala Ala Pro Ala
    530                 535                 540

Asp
545

<210>  6
<211>  566
<212>  PRT
<213>  Amino acid sequence of the endolevanase from Azotobacter chroococcum
DSM 2286

<400>  6

Met Asn Arg Asn Pro Thr Ser Met Arg Ala Ala Arg Arg Ala Ser Val
1               5                   10                  15

Cys Ala Leu Ala Ala Leu Leu Ala Ala Ala Thr Val Ala Val Phe Val
            20                  25                  30

Phe Ala Pro Ala Pro Ala Thr Pro Ser Gly Pro Glu Pro Ser Ser Pro
        35                  40                  45

Pro Leu Thr Lys Pro Ser Ala Phe Ser Arg Pro Ala Asp Trp Ser Pro
    50                  55                  60

Tyr Arg Pro Ala Val His Leu Thr Pro Ala Lys His Trp Met Asn Asp
65                  70                  75                  80

Pro Gln Arg Pro Ile Leu Ile Asp Gly Val Trp His Tyr Tyr Tyr Leu
                85                  90                  95

Tyr Asn Ala Asp Tyr Pro Lys Gly Asn Gly Thr Glu Trp Tyr His Ala
            100                 105                 110

Thr Ser Thr Asp Leu Val His Trp Gln Asp His Gly Val Ala Ile Asp
        115                 120                 125

Lys Tyr Lys Asn Gly Leu Gly Asp Ile Glu Thr Gly Ser Ala Val Ile
    130                 135                 140

Asp Thr Lys Asn Thr Ala Gly Phe Gly Ala Gly Ala Val Ile Ala Val
145                 150                 155                 160

Met Thr Gln Gln His Glu Gly Val Gln Arg Gln Ser Leu Phe Val Ser
                165                 170                 175

Thr Asp Gly Gly Tyr Arg Phe Lys Ala Tyr Asp Gly Asn Pro Val Met
            180                 185                 190

Asp Asn Pro Gly Ala Glu His Trp Arg Asp Pro Lys Ile Ile Trp Asp
        195                 200                 205

Asp Ala Arg Asp Glu Trp Leu Met Ala Leu Ala Glu Gly Asp Lys Ile
    210                 215                 220

Gly Phe Tyr Thr Ser Pro Asp Leu Lys Arg Trp Thr Tyr Arg Ser Gly
225                 230                 235                 240

Phe Glu Arg Asp Asp Leu Gly Ile Leu Glu Cys Pro Asp Leu Phe Arg
                245                 250                 255

Met Ser Val Asp Gly Asp Pro Ala Asn Thr Arg Trp Val Leu Ala Ala
            260                 265                 270

Gly Gly Asn Gly Ala Ser Lys Gly Met Thr Thr Gly Thr Val Tyr Trp
        275                 280                 285

Thr Gly Asn Trp Asp Gly Glu Arg Phe Thr Ala Asp Arg Asp Glu Pro
    290                 295                 300

Arg Trp Leu Asp Ser Gly Ala Asp Phe Tyr Ala Thr Val Thr Trp Asp
305                 310                 315                 320

Asp Pro Arg Gln Asp Asp Ala Glu Arg Leu Ala Ser Arg Tyr Ala Ile
                325                 330                 335

Gly Trp Leu Asn Asn Trp Ala Tyr Ala Thr Lys Leu Pro Thr Gly Asp
            340                 345                 350

Trp His Gly Gly Ala Asp Ser Ile Val Arg Arg Ile Glu Leu Arg Ser
        355                 360                 365

Val Asp Gly Glu Pro Leu Leu Val Ser Gln Pro Val Asp Ala Leu Asp
    370                 375                 380

Lys Leu Ala Gly Asn Ala Glu Val Arg Ser Lys Val Arg Val Thr Ala
385                 390                 395                 400

Ala Ser Gly Thr Thr Leu Pro Gln Pro Glu Ser Asp Ala Tyr Arg Leu
                405                 410                 415

Gln Val Glu Leu Asp Ala Ala Ser Gly Ala Asn Glu Val Arg Phe Arg
            420                 425                 430

Val Lys Glu Gly Gly Gly His Phe Thr Thr Val Gly Tyr Asn Phe Val
        435                 440                 445

His Gly Val Ala Phe Val Ala Arg Ala Ala Asp Ala Ala Ala Glu Arg
    450                 455                 460

Met Pro Ala Val Tyr Arg Glu Val Arg Thr Ala Pro Ala Pro Ala Arg
465                 470                 475                 480

Asp Gly Thr Val Thr Leu Asp Val Ile Val Asp Ala Ala Ser Val Glu
                485                 490                 495

Val Phe Val Asn Asp Gly Glu Ala Val Leu Ser Asn Leu Ala Phe Gly
            500                 505                 510

Ala Pro Gly Ala Asn Gly Leu Ser Val Glu Ser Leu Gly Gly Asp Thr
        515                 520                 525

Glu Leu Arg Ser Phe Arg Leu Thr Pro Leu Ala Ile Ala Pro Ile Glu
    530                 535                 540

Arg His Met Glu Gln Ala Pro Gln Gly Asn Gly Gly Ser Ala Gly Lys
545                 550                 555                 560

Pro Ala Ala Pro Ala Asp
                565

<210>  7
<211>  1638
<212>  DNA
<213>  Artificial

<220>
<223>  Nucleotide sequence of the modified endolevanase gene from
       Azotobacter chroococcum DSM 2286

<400>  7
atggctagct ggagccaccc gcagttcgaa aaaggcgccc ccgctccggc gaccccgtca      60

ggccccgagc cgtcgagccc gccgctgacc aagccctccg cattctcgcg gccggccgac     120

tggtcgccct accggcccgc ggtgcacctc acccccgcca agcactggat gaacgatccc     180

cagcgcccga tcctgatcga cggcgtctgg cactactact acctctacaa cgccgactac     240

cccaagggga acggcaccga gtggtaccac gcgacctcga ccgacctcgt gcactggcag 300

gatcacggcg tcgcgatcga caagtacaag aacggcctcg gcgacatcga gaccggcagc 360

gccgtgatcg acaccaagaa caccgcgggc ttcggcgccg gcgcggtgat cgccgtcatg 420

acccagcagc acgagggcgt gcaacggcag tcgctcttcg tctcgaccga cggcggctac 480

cgcttcaagg cgtacgacgg caaccccgtc atggacaacc cgggcgccga gcactggcgt 540

gacccgaaga tcatctggga cgacgcccgc gacgagtggc tgatggcgct cgccgagggc 600

gacaagatcg gtttctacac ttcgcccgac ctgaagcgct ggacgtaccg atcgggcttc 660

gagcgcgacg acctcggcat cctcgagtgc cccgacctgt tccgcatgtc ggtcgacggt 720

gacccggcga acacacgatg ggtgctcgcg gcaggcggga atggtgccag caagggcatg 780

accacgggaa ccgtgtactg gaccggcaac tgggacggcg agcgcttcac cgcggatcgc 840

gacgaaccgc gatggctcga ctcgggcgcc gacttctacg ccaccgtgac gtgggacgac 900

ccgcgccagg acgacgccga acgcctcgcc tcccgctacg ccatcggctg gctcaacaac 960

tgggcctacg ccaccaagct gccgaccggc gactggcacg gcggcgccga ctcgatcgtg 1020

cgacgcatcg agctgcgctc ggtcgacggc gagccgctgc tcgtctcgca gcccgtcgac 1080

gcgctcgaca agctcgcggg taatgccgag gtgcgctcca aggtacgggt gacggcagcc 1140

tcggggacga cgctccccca gccggaatcc gatgcctacc gactccaggt cgagctcgac 1200

gcggcctcgg gcgccaacga ggtgcgcttc cgcgtcaagg aaggtggcgg gcacttcacg 1260

accgtcgggt acaacttcgt gcacggcgtc gccttcgtcg cgcgcgccgc agatgctgcg 1320

gccgagcgga tgcccgcggt ctaccgcgag gttcgtacgg ccccggcgcc ggcgcgcgac 1380

ggcaccgtca cccttgacgt catcgtcgat gcggcctcgg tcgaggtctt cgtgaacgac 1440

ggcgaggccg tgctctcgaa tcttgcgttc ggggctcccg gcgcgaacgg gctctcggtc 1500

gagtcgctcg gcggcgacac cgaactgcgc tccttccgac tcacgccgct cgcgatcgcg 1560

ccgattgagc ggcatatgga gcaggccccc caaggcaacg gtggctcagc tggcaagcca 1620

gcggcgcccg cagactga 1638

<210> 8
<211> 1701
<212> DNA
<213> Nucleotide sequence of the endolevanase gene from Azotobacter
chroococcum DSM 2286

<400> 8
atgaaccgca atcccacctc gatgcgcgca gctcggcgcg catcggtctg cgcgctcgcc 60

gcgctgctcg ccgccgcaac cgtggcggtg ttcgtgttcg cgcccgctcc ggcgaccccg 120

tcaggccccg agccgtcgag cccgccgctg accaagccct ccgcattctc gcggccggcc 180

gactggtcgc cctaccggcc cgcggtgcac ctcacccccg ccaagcactg gatgaacgat 240

ccccagcgcc cgatcctgat cgacggcgtc tggcactact actacctcta caacgccgac 300

taccccaagg ggaacggcac cgagtggtac cacgcgacct cgaccgacct cgtgcactgg 360

caggatcacg gcgtcgcgat cgacaagtac aagaacggcc tcggcgacat cgagaccggc 420

agcgccgtga tcgacaccaa gaacaccgcg ggcttcggcg ccggcgcggt gatcgccgtc 480

atgacccagc agcacgaggg cgtgcaacgg cagtcgctct tcgtctcgac cgacggcggc 540

taccgcttca aggcgtacga cggcaacccc gtcatggaca acccgggcgc cgagcactgg 600

cgtgacccga agatcatctg ggacgacgcc cgcgacgagt ggctgatggc gctcgccgag 660

ggcgacaaga tcggtttcta cacttcgccc gacctgaagc gctggacgta ccgatcgggc 720

ttcgagcgcg acgacctcgg catcctcgag tgccccgacc tgttccgcat gtcggtcgac 780

ggtgacccgg cgaacacacg atgggtgctc gcggcaggcg ggaatggtgc cagcaagggc 840

atgaccacgg gaaccgtgta ctggaccggc aactgggacg gcgagcgctt caccgcggat 900

cgcgacgaac cgcgatggct cgactcgggc gccgacttct acgccaccgt gacgtgggac 960

gacccgcgcc aggacgacgc cgaacgcctc gcctcccgct acgccatcgg ctggctcaac 1020

aactgggcct acgccaccaa gctgccgacc ggcgactggc acggcggcgc cgactcgatc 1080

gtgcgacgca tcgagctgcg ctcggtcgac ggcgagccgc tgctcgtctc gcagcccgtc 1140

gacgcgctcg acaagctcgc gggtaatgcc gaggtgcgct ccaaggtacg ggtgacggca 1200

gcctcgggga cgacgctccc ccagccggaa tccgatgcct accgactcca ggtcgagctc 1260

gacgcggcct cgggcgccaa cgaggtgcgc ttccgcgtca aggaaggtgg cgggcacttc 1320

acgaccgtcg ggtacaactt cgtgcacggc gtcgccttcg tcgcgcgcgc cgcagatgct 1380

gcggccgagc ggatgcccgc ggtctaccgc gaggttcgta cggccccggc gccggcgcgc 1440

gacggcaccg tcacccttga cgtcatcgtc gatgcggcct cggtcgaggt cttcgtgaac 1500

gacggcgagg ccgtgctctc gaatcttgcg ttcggggctc ccggcgcgaa cgggctctcg 1560

gtcgagtcgc tcggcggcga caccgaactg cgctccttcc gactcacgcc gctcgcgatc 1620

gcgccgattg agcggcatat ggagcaggcc ccccaaggca acggtggctc agctggcaag 1680

ccagcggcgc ccgcagactg a 1701

<210> 9
<211> 31
<212> DNA
<213> Artificial

<220>
<223> p5_levS1417_for

<400> 9
attaccgcgg aaaatgctat ttccagccga a 31

<210>  10
<211>  30
<212>  DNA
<213>  Artificial

<220>
<223>  p5_levS1417_rev

<400>  10
attactcgag tcaggcacga acgtcatagg                                        30


<210>  11
<211>  42
<212>  DNA
<213>  Artificial

<220>
<223>  levS1417_EcoRV_for

<400>  11
attagatatc aggaggaaaa aaaaatgaat gctatttcca gc                          42


<210>  12
<211>  30
<212>  DNA
<213>  Artificial

<220>
<223>  levS1417_AscI_rev

<400>  12
attaggcgcg cctcaggcac gaacgtcata                                        30


<210>  13
<211>  36
<212>  DNA
<213>  Artificial

<220>
<223>  levB2286 _p5_f

<400>  13
atggtaggtc tcagcgcccc cgctccggcg accccg                                 36


<210>  14
<211>  37
<212>  DNA
<213>  Artificial

<220>
<223>  levB2286 _p5_r

<400>  14
atggtaggtc tcatatcagt ctgcgggcgc cgctggc                                37


<210>  15
<211>  47

<212>  DNA
<213>  Artificial

<220>
<223>  levS1417_Assembly_f

<400>  15
gacccgacac catcgaatgg attaattcct aatttttgtt gacactc                    47


<210>  16
<211>  38
<212>  DNA
<213>  Artificial

<220>
<223>  levS1417_Assembly_r

<400>  16
attaggaatt aatcatctgg agatccgtga cgcagtag                              38

## Claims

1. A method for preparing fructooligosaccharides comprising or consisting of the following steps:
(a) optionally introducing at least one nucleic acid molecule comprising a first nucleotide sequence which encodes a levansucrase and/or a second nucleotide sequence which encodes an endolevanase into at least one host organism;
(b) providing
(i) a host organism comprising a first nucleotide sequence which encodes a levansucrase and a second nucleotide sequence which encodes an endolevanase;
or
(ii) a first host organism comprising a first nucleotide sequence which encodes a levansucrase and a second host organism comprising the second nucleotide sequence which encodes an endolevanase;
(c) cultivating
(i) the host organism comprising the first nucleotide sequence which encodes a levansucrase and the second nucleotide sequence which encodes an endolevanase under conditions which allow the expression of the first and the second nucleotide sequence;
or
(ii) the first host organism comprising the first nucleotide sequence which encodes a levansucrase and the second host organism comprising the second nucleotide sequence which encodes an endolevanase under conditions which allow the expression of the first and the second nucleotide sequence; and
(d) preparing fructooligosaccharides using the levansucrase and the endolevanase expressed in step (c), by subjecting the enzymes to conditions which allow the production of the fructooligosaccharides.

2. The method according to claim 1,
wherein in step (d) the levansucrase and the endolevanase are, individually or together,
(i) comprised in a culture supernatant from the culture medium used in step (c), wherein the culture supernatant is subjected to conditions which allow the preparation of said fructooligosaccharides; and/or
(ii) comprised in a cell extract from the at least one host organism cultivated in step (c), wherein the cell extract is subjected to conditions which allow the preparation of said fructooligosaccharides; and/or
(iii) comprised in a host organism or at the surface of the host organism cultivated in step (c), wherein the host organism is subjected to conditions which allow the preparation of said fructooligosaccharides; and/or
(iv) provided in a purified or immobilized form.

3. The method according to claim 1 or 2, comprising or consisting of the following steps:
(a) optionally introducing one nucleic acid molecule comprising a first nucleotide sequence which encodes a levansucrase and a second nucleotide sequence which encodes an endolevanase into a host organism;
(b) providing a host organism comprising the first nucleotide sequence which encodes a levansucrase and the second nucleotide sequence which encodes an endolevanase;
(c) cultivating the host organism comprising the first nucleotide sequence which encodes a levansucrase and the second nucleotide sequence which encodes an endolevanase under conditions which allow the expression of the first and the second nucleotide sequence; and
(d) preparing fructooligosaccharides using the levansucrase and the endolevanase expressed in step (c) and comprised in the cell extract from the host organism or in the culture supernatant from the culture medium after step (c), by subjecting said cell extract or culture supernatant to conditions which allow the production of said fructooligosaccharides.

4. The method according to any one of claims 1 to 3, wherein the fructooligosaccharides obtained in step (d) comprise, essentially consist of or consist of compounds of the formulas Fₘ and/or GFₙ, wherein
F is a monomeric fructose unit, preferably a D-fructose unit;
G is a monomeric glucose unit, preferably a D-glucose unit;
m is ≥3, preferably 3 to 20, more preferably 3 to 15, more preferably 3 to 13, most preferably 3 to 10;
n is ≥2, preferably 2 to 19, more preferably 2 to 14, more preferably 2 to 12, most preferably 2 to 9; m and/or n may be different in the individual fructooligosaccharides obtained in step (d).

5. The method according to any one of claims 1 to 4, wherein
the levansucrase comprises or consists of
(i) an amino acid sequence set forth in SEQ ID Nos. 1 or 2; or
(ii) an amino acid sequence, which has at least 60 %, 70 %, 75 %, 80 %, 85 %, 90 %, 91 %, 92 %, 93 %, 94 %, 95 %, 96 %, 97 %, 98 % or 99 % sequence identity with the amino acid sequence set forth in SEQ ID Nos. 1 or 2 over the full length of the sequence; or
(iii) a fragment of (i) or (ii);
and/or
wherein the endolevanase comprises or consists of
(i) an amino acid sequence set forth in SEQ ID Nos. 5 or 6; or
(ii) an amino acid sequence, which has at least 60 %, 70 %, 75 %, 80 %, 85 %, 90 %, 91 %, 92 %, 93 %, 94 %, 95 %, 96 %, 97 %, 98 % or 99 % sequence identity with the amino acid sequence set forth in SEQ ID Nos. 5 or 6 over the full length of the sequence; or
(iii) a fragment of (i) or (ii).

6. The method according to any one of claims 1 to 5, wherein the conditions which allow the preparation of said fructooligosaccharides comprise providing sucrose to the levansucrase and endolevanase used in step (d),
wherein sucrose is converted to the fructooligosaccharides by levansucrase and endolevanase catalysis,
wherein the levansucrase hydrolyzes the sucrose to form fructan-polymers,
and then the endolevanase hydrolyzes the fructan-polymers to prepare the fructooligosaccharides obtained in step (d).

7. The method according to any one of claims 1 to 6, wherein the host organism is a bacterial or yeast organism, preferably a bacterial organism, more preferably *Escherichia coli* or a *Gluconobacter* species, most preferably *Escherichia coli* BL21 or *Gluconobacter japonicus* LMG 1417.

8. The method according to any one of claims 1 to 7, wherein the first nucleotide sequence which encodes the levansucrase and/or the second nucleotide sequence which encodes the endolevanase is
(i) comprised in an expression vector; and/or
(ii) integrated in the genome, preferably in the chromosomal DNA, of the host organism.

9. Fructooligosaccharides obtainable by the method according to any one of claims 1 to 8.

10. An expression vector comprising a first nucleotide sequence which encodes a levansucrase and/or a second nucleotide sequence which encodes an endolevanase;
wherein the first nucleotide sequence which encodes the levansucrase comprises or consists of a nucleotide sequence
(i) set forth in SEQ ID Nos. 3 or 4; or
(ii) which has at least 70 %, 75 %, 80 %, 85 %, 90 %, 91 %, 92 %, 93 %, 94 %, 95 %, 96 %, 97 %, 98 % or 99 % sequence identity with the nucleotide sequence set forth in SEQ ID Nos. 3 or 4 over the full length of the sequence;
and/or
wherein the second nucleotide sequence which encodes the endolevanase comprises or consists of a nucleotide sequence
(i) set forth in SEQ ID Nos. 7 or 8; or
(ii) which has at least 70 %, 75 %, 80 %, 85 %, 90 %, 91 %, 92 %, 93 %, 94 %, 95 %, 96 %, 97 %, 98 % or 99 % sequence identity with the nucleotide sequence set forth in SEQ ID Nos. 7 or 8 over the full length of the sequence.

11. A genetically modified host organism comprising
(i) the expression vector as defined in claim 10; and/or
(ii) the first nucleotide sequence which encodes the levansucrase and/or the second nucleotide sequence which encodes the endolevanase as defined in any one of the preceding claims in its genome, preferably in its chromosomal DNA; and/or
(iii) the levansucrase and/or the endolevanase as defined in any one of the preceding claims.

12. A cell extract or a culture supernatant comprising the levansucrase and/or the endolevanase as defined in any one of the preceding claims.

13. A prebiotic or food supplement comprising or (essentially) consisting of the fructooligosaccharides as defined in claim 9.

14. A method for preparing levan comprising or consisting of the following steps:
(a) optionally introducing at least one nucleic acid molecule comprising a first nucleotide sequence which encodes a levansucrase into a host organism;
(b) providing a host organism comprising a first nucleotide sequence which encodes a levansucrase;
(c) cultivating the host organism under conditions which allow the expression of the first nucleotide sequence;and
(d) preparing levan using the levansucrase expressed in step (c) and subjecting it to conditions which allow the production of levan.

15. The method according to claim 14, wherein in step (d) the levansucrase is
(i) comprised in a culture supernatant from the culture medium used in step (c), wherein the culture supernatant is subjected to conditions which allow the preparation of levan; or
(ii) comprised in a cell extract from the host organism cultivated in step (c), wherein the cell extract is subjected to conditions which allow the preparation of levan; or
(iii) comprised in a host organism or at the surface of the host organism cultivated in step (c), wherein the host organism is subjected to conditions which allow the preparation of levan; or
(iv) provided in a purified or immobilized form.
